# NEW EUROPEAN PATENT SPECIFICATION

(11) **EP 0 608 359 B2**
(45) Date of publication and mention of the opposition decision: **09.12.2009**
(45) Mention of the grant of the patent: 28.07.1999
(21) Application number: 92922535.7
(22) Date of filing: 26.10.1992
(51) Int. Cl.: A01H 5/10

(54) **SOYBEAN PRODUCTS WITH IMPROVED CARBOHYDRATE COMPOSITION AND SOYBEAN PLANTS**
SOJAPRODUKTE MIT VERBESSERTEM KOHLEHYDRATGEHALT UND SOJAPFLANZEN
PRODUITS DU SOYA AVEC COMPOSITION AMELIOREE EN HYDRATES DE CARBONE

(30) Priority: 24.10.1991 US 782033
(43) Date of publication of application: 03.08.1994
(73) Proprietor: E.I. DU PONT DE NEMOURS AND COMPANY, Wilmington, DE 19898 (US)
(72) Inventor: KERR, Phillip, S., Newark, DE 19714 (US)
(74) Representative: Jones, Elizabeth Louise
(86) International application number: PCT/US1992/008958
(87) International publication number: WO 1993/007742

(56) References cited:
- US-A- 4 371 556
- US-A- 4 454 804
- US-A- 5 710 365
- AGRICULTURAL AND BIOLOGICAL CHEMISTRY vol. 49, no. 4, 1985, pages 933 - 937 T.YASUI 'Dissimilarity in low molecular weight carbohydrate composition of the seeds of cultivated soybean (Glycinen max (L.) Merrill subsp. max) and wild soybean (G.max subsp.soja (Sieb.et Zucc.) Ohashi.)', XP002914381
- H.E.SNYDER & T.W.KWON 'Soybean utilization' 1987 , VAN NOSTRAND REINHOLD COMP. , NEW YORK Chapter 2 pages 58-59,paragraph Soluble carbohydrates; Chapter 3, pages 74-94, Processing of soybeans, XP002191359
- AGRONOMY JOURNAL vol. 64, no. 5, 1972, pages 613 - 616 T.HYMOWITZ ET AL 'Relationship between the content of oil, protein and sugar in soybeen seed', XP002035319, cited in the application
- ACTA AGROBOTANICA vol. 36, no. 1-2, 1983, pages 41 - 48 B.JACÔRZYNSKI & N.BARYLKO-PIKIELNA 'Quantitative and qualitative characteristics of sugars in the seeds of soybean cultivated in Poland', XP008034396, cited in the applicatio n
- SOIL SCIENCE AND PLANT ANALYSIS vol. 3, 1972, pages 367 - 373 T.HYMOWITZ ET AL 'Stability of sugar content in soybean strains' XP008034397, cited in the application
- J.M.POEHLMAN 'Breeding fieldcrops' 1986 , VAN NOSTRAND REINHOLD , NEW YORK US Chapter 17, pages 421-450, "Breeding soybeans", XP001173068
- Y.P.S.BAJAJ -ed- : "Biotechnology in agriculture and forestry" Vol.2 Crops I. 1986 Springer Verlag BERLIN; chapter II.1 pages 283-308; D.F.HILDEBRAND et al : "Soybean (Glycine max(L.) Merr.)", XP000648050

## Description

### FIELD OF THE INVENTION

This invention relates to soybean protein products having significantly lower stachyose content as a function of using the seeds of a soybean line having a heritable seed phenotype of less than 30 µmol/g of seed (as is). The present invention also relates to such low stachyose containing soybeans.

### BACKGROUND OF THE INVENTION

Raffinose saccharides are a group of D-Galadose-containing oligosaccharides of sucrose that are widely distributed in plants. Raffinose saccharides are characterized by having the general formula:
[0-β-D-galactopyranosyl-(1→6)ₙ-α-glucopyranosyl-(1→2)-β-D-fructofuranoside where n=0 through n=4 are known respectively as sucrose, raffinose, stachyose, verbascose, and ajugose.

Extensive botanical surveys of the occurrence of raffinose saccharides have been reported in the scientific literature [see Dey (1985) in Biochemistry of Storage Carbohydrates in Green Plants, Academic Press, London, pp. 53-129]. Raffinose saccharides are thought to be second only to sucrose among the nonstructural carbohydrates with respect to abundance in the plant kingdom. In fact, raffinose saccharides may be ubiquitous, at least among higher plants. Raffinose saccharides accumulate in significant quantities in the edible portion of many economically significant crop species. Examples include soybean (*Glycine max* L. Merrill), sugar beet *(Beta vulgaris),* cotton *(Gossypium hirsutum* L), canola (*Brassica* sp.) and all of the major edible leguminous crops including beans *(Phaseolus* sp.), chick pea *(Cicer arietinum),* cowpea *(Vigna unguiculata),* mung bean *(Vigna radiata),* peas *(Pisum sativum),* lentil *(Lens culinaris)* and lupine *(Lupinus* sp.).

The biosynthesis of raffinose saccharides has been fairly well characterized [see Dey (1985) in Biochemistry of Storage Carbohydrates in Green Plants]. The committed reaction of raffinose saccharide biosynthesis involves the synthesis of galactinol (O-α-D-galactopyranosyl-(1→1)-myo-inositol) from UDPgalactose and myo-inositol. The enzyme that catalyzes this reaction is galactinol synthase. Synthesis of raffinose and higher homologues in the raffinose saccharide family from sucrose is thought to be catalyzed by distinct galactosyltransferases (e.g., raffinose synthase, stachyose synthase, etc.).

Although abundant in many species, raffinose saccharides are an obstacle to the efficient utilization of some economically important crop species. Raffinose saccharides are not digested directly by animals, primarily because α-galactosidase is not present in the intestinal mucosa [Gitzelmann and Auricchio (1965) Pediatrics 36:231-236, Rutloff et al. (1967) Nahrung 11:39-46]. However, microflora in the lower gut are readily able to ferment the raffinose saccharides which results in an acidification of the gut and production of carbon dioxide, methane and hydrogen [Murphy et al. (1972) J. Agr. Food Chem. 20:813-817, Cristofaro et al. (1974) in Sugars in Nutrition, Ch 20, 313-335, Reddy et al. (1980) J. Food Science 45:1161-1164). The resulting flatulence can severely limit the use of leguminous plants in animal, including human, diets. It is unfortunate that the presence of raffinose saccharides restricts the use of soybeans in animal, including human, diets because otherwise this species is an excellent source of protein and fiber.

The soybean is well-adapted to machinery and facilities for harvesting, storing and processing that are widely available in many parts of the world. In the U.S. alone, approximately 28 million metric tons of meal were produced in 1988 (Oil Crops Situation and Outlook Report, Apr. 1989, U.S. Dept. of Agriculture, Economic Research Service). Typically, hulls are removed and then the oil is extracted with hexane in one of several extraction systems. The remaining defatted flakes can then be used for a variety of commercial soy protein products [see Soy Protein Products, Characteristics, Nutritional Aspects and Utilization (1987) Soy Protein Council]. Foremost among these in volume of use is soybean meal, the principle source of protein in diets used for animal feed, especially those for monogastric animals such as poultry and swine.

Although the soybean is an excellent source of vegetable protein, there are inefficiencies associated with its use that appear to be due to the presence of raffinose saccharides. Compared to maize, the other primary ingredient in animal diets, gross energy utilization for soybean meal is low [see Potter and Potchanakorn (1984) in Proceedings World Soybean Conference III, 218-224]. For example, although soybean meal contains approximately 6% more gross energy than ground yellow corn, it has about 40 to 50% less metabolizable energy when fed to chickens. This inefficiency of gross energy utilization does not appear to be due to problems in digestion of the protein fraction of the meal, but rather due to the poor digestion of the carbohydrate portion of the meal. It has been reported that removal of raffinose saccharides from soybean meal by ethanol extraction results in a large increase in the metabolizable energy for broilers [Coon et al. (1988) Proceedings Soybean Utilization Alternatives, University of Minnesota, 203-211].
Removal of the raffinose saccharides was associated with increased utilization of the cellulosic and hemicellulosic fractions of the soybean meal.

A variety of processed vegetable protein products are produced from soybean. These range from minimally processed, defatted items such as soybean meal, grits, and flours to more highly processed items such as soy protein concentrates and soy protein isolates. In other soy protein products the oil is not extracted, full-fat soy flour for example. In addition to these processed products, there are also a number of speciality products based on traditional Oriental processes, which utilize the entire bean as the starting material. Examples include soy milk, soy sauce, tofu, natto, miso, tempeh, and yuba.

Examples of use of soy protein products in human foods include soy protein concentrates, soy protein isolates, textured soy protein, soy milk, and infant formula. Facilities and methods to produce protein concentrates and isolates from soybeans are available across the world. One of the problems faced by producers of soy protein concentrates and isolates is the challenge of selectively purifying the protein away from the raffinose saccharides. Considerable equipment and operating costs are incurred as a result of removing the large amounts of raffinose saccharides that are present in soybeans.

The problems and costs associated with raffinose saccharides could be reduced or eliminated through the availability of genes that confer a reduction of raffinose saccharide content of soybean seeds. Such genes could be used to develop soybean varieties having inherently reduced raffinose saccharide content. Soybean varieties with inherently reduced raffinose saccharide content would improve the nutritional quality of derived soy protein products and reduce processing costs associated with the removal of raffinose saccharides. Said low raffinose saccharide soybean varieties would be more valuable than conventional varieties for animal and human diets and would allow mankind to more fully utilize the desirable nutritional qualities of this edible legume.

Efforts have been made to identify soybean germplasm that may contain genes that confer a low seed raffinose saccharide content phenotype. Surveys of the soybean germplasm collection, including Glycine max. Glycine soja, and Glycine hirsutum, tentatively identified PI lines that seemed to offer the potential for reducing raffinose saccharide content via conventional breeding [see Hymowitz, et al. (1972) Comm. In Soil Science and Plant Analysis 3:367-373, Hymowitz et al. (1972) Agronomy J. 64:613-616, Hymowitz and Collins (1974) Agronomy J. 66:239-240, Openshaw and Hadley (1978) Crop Science 18:581-584, Openshaw and Hadley (1981) Crop Science 21:805-808, and Saravitz (1986) Ph.D. Thesis, North Carolina State University, Horticultural Science Department]. However, when assayed under identical analytical conditions, none of the lines suggested in these prior surveys proved to be significantly lower in raffinose saccharide content than the currently available elite soybean lines. The primary reason for this may be due to the instability of the low raffinose saccharide phenotype. Results from germplasm collection surveys are highly influenced by the quality of the seed obtained from the collection. This is particularly true for raffinose saccharides in that seed carbohydrate composition has been shown to be influenced by seasonal, genetic and environmental factors [Jacorzynski and Barylko-Pikielna, (1983) Acta Agrobotanica 36:41-48, Saravitz (1986) Ph.D. Thesis, North Carolina State University, Horticultural Science Department]. Furthermore, seed storage conditions prior to analysis can also influence the composition [Ovacharov and Koshelev (1974) Fiziol. Rast. 21:969-974, Caffrey et al. (1988) Plant Physiol. 86:754-758, Schleppi and Burns (1989) Iowa Seed Science 11:9-12]. As a result, the potential exists for falsely identifying soybean germplasm whose reduced raffinose saccharide content is not heritable, but rather due to the environment in which the seeds were produced or stored prior to analysis. Collectively, these factors have severely limited efforts to identify soybean genes that reduce raffinose saccharide content.

The difficulty and unreliability of screens for raffinose saccharide content is reflected by the paucity of publicly available soybean carbohydrate data as compared to protein and oil quality data. For example, the USDA has numerous publications revealing the protein and oil quality contents for almost all (ca. 14,000) of the soybean PI lines in the USDA collection. However, although raffinose saccharide content is known to be a serious problem in soybeans, very little of the PI collection has actually been screened for this trait.

Demonstration of the stability of a low raffinose saccharide phenotype in subsequent generations (heritability of the phenotype) is required if the germplasm is to be of any utility in improving seed quality. It is therefore essential that any putative germplasm source be regrown to obtain fresh seed and reassayed (with appropriate lines as experimental controls) before it is declared as a potential source of low raffinose saccharide genes. Once the heritability (stability) of the phenotype is demonstrated, it is desirable to determine the inheritance (number and nature of genes that are involved) of the low raffinose saccharide phenotype. Inheritance information is extremely valuable for attempts to breed new soybean varieties that contain the low raffinose saccharide trait.

In light of the above described factors, it is apparent that soybean plants with heritable, substantially reduced raffinose saccharide content useful for preparing soy protein products with an improved carbohydrtae content are needed. Heretofore, the only means to acheive a desirable raffinose saccharide content was to physically and/or chemically treat the soybean.

### SUMMARY OF THE INVENTION

The present Invention comprises soybean according to claim 1.

A further embodiment of the invention is a method of using a soybean line as claimed in claim 1 having a genotype at the Stc 1 locus that confers a phenotype of a seed stachyose content of less than 30 µmol/g (as is), the method comprising processing said seeds to obtain a desired soy protein product. A further embodiment of the invention is a method of making a soy protein product comprising processing seeds of a soybean line as claimed in claim 1 having a heritable phenotype of a seed stachyose content of less than 30 µmol/g (as is). Preferred embodiments are methods of making a soy protein product comprising processing seed of a soybean line as claimed in claim 1 having a genotype at the Stc1 locus phenotype that confers a seed stachyose content of less than 30 µmol/g (as is).

The present invention further comprises methods for making a full fat soy protein product, the method comprising: (a) cracking seeds from a soybean line as claimed in claim 1 having a heritable phenotype of a seed stachyose content of less than 45 µmol/g (as is) to remove the meats from the hulls; (b) flaking the meats obtained in step a to obtain a desired flake thickness; (c) heat-denaturing the flakes obtained in step (b) to obtain a desired Nitrogen Solubility Index; and (d) grinding the denatured flakes of step (c) to obtain a desired particle size. The present invention further comprises adding soybean hulls to the product of step (c) to obtain a full fat soy protein product having a maximum fibre content of 7% at a moisture content of 12%.

The present invention further comprises a method of making a defatted soy protein product comprising: (a) cracking seeds from a soybean line as claimed in claim 1 having a heritable phenotype of a seed stachyose content of less than 30 µmol/g (as is) to remove the meats from the hulls; (b) flaking the meats obtained in step (a) to obtain a desired flake thickness: (c) contacting the full fat flakes obtained in step (b) with a solvent to extract oil from the flakes to a desired content level; (d) heat-denaturing the defatted flakes obtained in step (c) to obtain a desired Nitrogen Solubility Index; and (e) grinding the denatured, defatted flakes obtained in step (d) to obtain a desired particle size. The present invention further comprises adding soybean hulls to the product of step (c) to obtain a full fat soy protein product having a maximum fibre content of 7% at a moisture content of 12%. The heat-denaturing may be accomplished by flash desolventization. Extruding the full fat soy protein product or the defatted soy protein product to texturize or structure the product after the grinding step is also included in the present invention.

The present invention further comprises a method of making a soy protein concentrate product comprising: (a) cracking seeds from a soybean line as claimed in claim 1 having a heritable phenotype of a seed stachyose content of less than 30 µmol/g (as is) to remove the meats from the hulls; (b) flaking the meats obtained in step (a) to obtain a desired flake thickness: (c) contacting the full fat flakes obtained in step (b) with a first solvent to extract oil from the flakes to a desired oil content level; (d) contacting the detailed flakes obtained in step (c) with a second solvent to obtain a soy protein concentrate product with a protein content (6.25 x N) of not less than 65% (db). A preferred embodiment of this invention uses an aqueous alcohol solution from 55% to 90% as a second solvent. the soy protein concentrate product obtained in step (d) having a protein content (6.25 x N) of not less than 70%(db). A second preferred embodiment of this invention uses an acidic solution of pH 4 to pH 5 as a second solvent.

The present invention further comprise a method of making an isoelectric soy protein isolate product comprising: (a) cracking seeds from a soybean line as claimed in claim 1 having a heritable phenotype of a seed stachyose content of less than 30 µmol/g (as is) to remove the meats from the hulls; (b) flaking the meats obtained in step (a) to obtain a desired flake thickness; (c) contacting the full fat flakes obtained in step (b) with a first solvent to extract oil from the flakes to a desired oil content level; (d) contacting the defatted flakes obtained in step (c) with an aqueous solution of pH 8 to pH 9; (e) separating the soluble and insoluble fractions of the product of step d by physical means; (f) adjusting the pH of the soluble fraction obtained in step (e) to obtain a protein precipitate; (g) separating the protein precipitates of step (f) from the soluble fraction by physical means to obtain a soy protein isolate; (h) washing the product of step (g) with water; and (i) spray-drying the washed product of step (h) to obtain an isoelectric soy protein isolate product. A further embodiment of this invention comprises mixing the soy protein isolate product obtained in step (i) with sufficient alkali to increase the solubility of the product to a desired level.

A present invention further comprises a method of making a pet food product comprising: (a) combining farinaceous materials, proteinaceous material comprising a soy protein product from a soybean as claimed in claim 1 with a stachyose content of less than 45 µmol/g (as is) at an inclusion rate of less than 41%, animal fat, vitamins, minerals, and salt into a mixture; (b) extruding the mixture of step (a) through a die at an elevated temperature and pressure; (c) portioning the extruded mixture of step (b) into pieces of a desirable size; and (d) drying the products of step (c) to a desirable moisture content preferably a moisture content of less than 10%.

Also described is a full fat soy protein product from a soybean as claimed in claim 1 having a seed stachyose content respectively of less than 30 µmol/g (as is), preferably less than 35 µmol/g (as is) or more preferably less than 15 µmol/g (as is). The present invention provides a soy protein product made from a soybean as defined in any one of claims 1 to 4, wherein said product is soy milk having a stachyose content of less than 45 µmol/g (as is). A further embodiment of the invention also contains a protein content of greater than 42% at each of the stachyose content levels stated.

The present invention further comprises an undenatured, defatted soy protein product from a soybean as claimed in claim 1 having a stachyose content as claimed in claim 15. The invention further includes an undenatured, defatted soy protein product from a soybean as claimed in claim 1 and use product having a seed stachyose content preferably less than 35 µmol/g (as is) or more preferably less than 15 µmol/g (as is).

The present invention further comprises a heat-processed; defatted, desolventized and toasted soy protein product from a soybean as claimed in claim 1 having (a) a true metabolizable energy (TME_{N}) content of greater than 2850 Kcal/Kg (db); and (b) a stachyose content as claimed in claim 16. The invention further includes heat-processed, defatted, desolventized and toasted soy protein product from a soybean as claimed in claim 1 having a seed stachyose content preferably less than 35 µmol/g (as is) or more preferably less than 15 µmol/g (as is). A further embodiment of the invention also contains a protein content of greater than 51.5% at each of the stachyose content levels stated.

The present invention further comprises a heat-processed, defatted, flash-desolventized soy protein product from a soybean as claimed in claim 1 having a seed stachyose content of less than 45 µmol/g (as is). A further embodiment of the invention also contains a protein content of greater than 51.5% at each of the stachyose content levels stated.

The present invention further comprises a soy protein contentrate product having a protein content (6.5 x N) of not less than 65% (db) produced by the method comprising: (a) cracking seeds from a soybean line as claimed in claim 1 having a heritable phenotype of a seed stachyose content of less than 30 µmol/g (as is) to remove the meats from the hulls: (b) flaking the meats obtained in step (a) to obtain a desired flake thickness; (c) contacting the full fat flakes obtained in step (b) with a first solvent to extract oil from the flakes to a desired oil content level; (d) contacting the defatted flakes obtained in step (c) with a second solvent to obtain a soy protein concentrate product with a protein content (6.25 x N) of not less than 65% (db).

The present invention further comprises a pet food product having a soybean inclusion rate of between 25% and 41% and a total stachyose content of less than 10 µmol/g (db) wherein said soybean is as defined in any one of claims 1 to 4.

A further aspect of the invention is a method of using a soybean line having a genotype at the Stc1 locus that confers a phenotype of a seed stachyose content of less than 30 µmol/g (as is) to produce progeny lines, the method comprising:
(a) crossing a soybean plant comprising a stc1x allele with an agronomically elite soybean parent which does not comprise said allele, to yield a F1 hybrid;
(b) selfing the F1 hybrid for at least one generation; and
(c) identifying the progeny of step (b) homozygous for the stc1x gene and capable of producing seed having a stachyose content of less than 30 µmol/g (as is).

### BRIEF DESCRIPTION OF THE DRAWINGS

Figure 1 (A-D) show the distribution of total α-galactoside content in four segregating F2 populations resulting from the cross between LR28 and four agronomically elite lines. The figures graphically support the levels of total raffinose saccharides ["less than 110 µmol/g (as is)]".
Figures 2 (A-D) show the distribution of "low" seed stachyose content in four segregating F2 populations resulting from the cross between LR28 and four agronomically elite lines. The figures graphically support the levels of seed stachyose ["less than 45 µmol/g (as is)"].
Figure 3 shows a unimodal distribution of F2 phenotypes resulting from the LR28*LR484 cross. None of the F2 plants produced seed in the α-galactoside range of conventional soybean plants.

### DETAILED DESCRIPTION OF THE INVENTION

The present invention provides soybean genes, stc1a and stc1b, that confer the trait of improved seed carbohydrate composition to soybean plants. Through breeding techniques. Applicants' improved seed carbohydrate trait can be combined with any other desirable seed or agronomic trait. Examples of other desirable traits include, but are not limited to, high seed protein content and high seed yield. Processing of seeds from soybean lines containing stc1a or stc1b will produce soy protein products with reduced raffinose saccharide content and improved metabolizable energy and thus possess added value for individuals who either produce soy protein products for animal, including human, food uses or use soy protein products as major components in the diets for themselves or their animals.

In the context of this disclosure, a number of terms shall be utilized. As used herein, "soybean" refers to the species *Glycine max, Glycine soja,* or any species that is sexually cross compatible with *Glycine max.* A "line" is a group of plants of similar parentage that display little or no genetic variation between individuals for a least one trait. Such lines may be created by one or more generations of self-pollination and selection, or vegetative propagation from a single parent including by tissue or cell culture techniques. "Germplasm" refers to any plant(s), line(s), or population of plants that has/have the potential to be used as parent(s) in a plant breeding program. As used herein, "PI" or "plant introduction" refers to one of many soybean germplasm lines collected and maintained by the the United States Department of Agriculture. "Agronomic performance" or "agronomics" refers to heritable crop traits such as good emergence, seedling vigor, vegetative vigor, adequate disease tolerance and, ultimately, high seed yield. "Seed yield" or "yield" refers to productivity of seeds per unit area (e.g., bushels/acre or metric tons/hectare) that a particular soybean line is capable of producing in a specific environment or generally in many environments. An "agronomically elite line" or "elite line" refers to a line with desirable agronomic performance that may or may not be used commercially. A "variety", "cultivar", "elite variety", or "elite cultivar" refers to an agronomically superior elite line that has been extensively tested and is (or was) being used for commercial soybean production. "Mutation" refers to a detectable and heritable genetic change (either spontaneous or induced) not caused by segregation or genetic recombination. "Mutant" refers to an individual, or lineage of individuals, possessing a mutation. A "population" is any group of individuals that share a common gene pool. In the instant invention, this includes M1, M2, M3, M4, F1, and F2 populations. As used herein, an "M1 population" is the progeny of seeds (and resultant plants) that have been exposed to a mutagenic agent, while "M2 population" is the progeny of self-pollinated M1 plants, "M3 population" is the progeny of self-pollinated M2 plants, and "M4 population" is the progeny of self-pollinated M3 plants. As used herein, an "F1 population" is the progeny resulting from cross pollinating one line with another line. The format used herein to depict such a cross pollination is "female parent*male parent". An "F2 population" is the progeny of the self-pollinated F1 plants. An "F2-derived line" or "F2 line" is a line resulting from the self-pollination of an individual F2 plant. An F2-derived line can be propagated through subsequent generations (F3, F4, F5 etc.) by repeated self-pollination and bulking of seed from plants of said F2-derived line. A "pedigree" denotes the parents that were crossed to produce the segregating population from which a given line was selected. For example, a pedigree of A*B for a given line C indicates A and B are the parents of C. Although dines of similar pedigree may have a trait in common (due to selection for said trait), said lines of similar pedigree may be quite different in terms of other traits. "Heritability" is a relative term referring to the extent to which a given phenotype is determined by genetic factors as opposed to environmental or analytical error factors. An "environment" is used to define a specific time, general geographical area, and climatic conditions in which soybean plants were grown to produce seeds. Within the context of this application, soybean seeds produced in a common environment were seeds that were produced on plants that were planted during the same day, within the same 1 km radius, and under similar growing conditions. Environments within this application are identified by the year and geographical site at which seeds were produced. A "heritable trait" refers to a phenotype that is largely determined by genetic factors and is relatively stable and predictable over many environments. "Heritability" does not necessarily imply that said genetic factors have been characterized. "Inheritance" refers to the actual number and nature of genes that confer a given heritable trait. For example, Mendelian segregation patterns are used to deduce the "inheritance" of a trait. "Inherent" is used to denote a plant material or seed characteristic that is conferred by the genetic makeup of the plant as opposed to the environment in which the plant was grown or the way that the plant material or seed was stored or processed. Since raffinose saccharide content of soybean seeds is known to decrease with weathering and aging of seeds [Ovacharov and Koshelev (1974) Fiziol. Rast. 21:969-974, Caffrey et al. (1988) Plant Physiol. 86:754-758, Schleppi and Burns (1989) Iowa Seed Science 11:9-12], all claims regarding the heritable carbohydrate content of seeds in the current application are in reference to the carbohydrate content of seeds that have been stored for less than one year at 1 to 27°C and at 0 to 70% relative humidity.

As used herein, "total α-galactoside" content refers to all seed α-linked carbohydrate soluble in the solvent system described herein and is capable of being assayed using the α-galadosidase/galactose dehydrogenase method described herein. "Total raffinose saccharides" refers to the seed α-galactose content soluble in the solvent system described herein and represented by the sum of stachyose (2 moles α-galactose/mole), raffinose (1 mole α-galactose/mole) and galactinol (1 mole α-galactose/mole) as determined by methods described herein. "Raffinose saccharide content" is a general term referring to the seed raffinose saccharide content as determined by any type of analytical method. The term "as is" refers to the basis (i.e., moisture content at the time of analysis, as determined by AOCS Method Ba 2a-38, of a given seed or soy protein product) used to express the units of carbohydrate content. The range in moisture content of materials whose carbohydrate content was expressed on an as is basis was from 6 to 13%. The term "dry basis" or "(db)" refers to the moisture content of materials that have been placed in a 45°C oven until they have reached contant weight.

The term "Stc1 locus" refers to a genetic locus within soybean that affects raffinose saccharide content in soybean seeds. The term "Stc1" (with a capital "S") refers to the wild type allele that confers a normal raffinose saccharide content. The terms "stc1a" and "stc1b" (with a lower case "s") refer to two separate but allelic soybean genes at the Stc1 locus, that confer low raffinose saccharide content. The term "stc1x" (lower case "s") is a general term referring to any allele at the Stc1 locus (including stc1a, stc1b, and other possible alleles) that confer a low total raffinose saccharide phenotype. "LR28" (an abbreviation synonymous with "PI 200.508") is the designation for a soybean line that was the source of the sty1a gene discovered by Applicants. "LR484" is the designation for a soybean line derived from mutagenesis of elite cultivar "Williams 82". LR484 is the source of gene "stc1b" discovered by Applicants. The phrase "line(s) containing stc1a" or "stc1a line(s)" indicates that the line(s) is homozygous for stc1a as evidenced by the line's pedigree and abnormally low raffinose saccharide content. The phrase "line(s) containing stc1b" or "stc1b line(s)" indicates that the line(s) is homozygous for stc1b as evidenced by the line's pedigree and abnormally low raffinose saccharide content. The phrase "lines containing stc1x" or "stc1x line(s)" indicates that the line(s) is homozygous for stc1x as evidenced by the line's pedigree and abnormally low raffinose saccharide content. "Conventional soybean lines" refers to lines that do not contain an stc1x allele.

"Soy protein products" are defined as those items produced from soybean seed used in feeds or foods and include, but are not limited to, those items listed in Table 1.

**TABLE 1**

| Soy Protein Products Derived from Soybean Seeds^{a} | |
|---|---|
| Whole Soybean Products | Processed Soy Protein Products |
| Roasted Soybeans | Soybean Meal |
| Baked Soybeans | Soy Grits |
| Soy Sprouts | Full Fat and Defatted Flours |
| Soy Milk | Soy Protein Isolates |
| | Soy Protein Concentrates |
| Speciality Soy Foods/Ingredients | Textured Soy Proteins |
| Soy Milk | Textured Flours and Concentrates |
| Tofu | Structured Concentrates |
| Tempeh | Structured Isolates |
| Miso | |
| Soy Sauce | |
| Hydrolyzed Vegetable Protein | |
| Whipping Protein | |

| | |
|---|---|
| ^{a}See Soy Protein Products: Characteristics, Nutritional Aspects and Utilization (1987). Soy Portein Council | |

"Processing" refers to any physical and chemical methods used to obtain the products listed in Table 1 and includes, but is not limited to heat conditioning, flaking and grinding, extrusion, solvent extraction, or aqueous soaking and extraction of whole or partial seeds. Furthermore, "processing" includes the methods used to concentrate and isolate soy protein from whole or partial seeds, as well as the various traditional Oriental methods in preparing fermented soy food products. Trading Standards and Specifications have been established for many of these products (see National Oilseed Processors Association Yearbook and Trading Rules 1991-1992). Products referred to as being "High" or "Low" protein are those as decribed by these Standard Specifications. "NSI" refers to the Nitrogen Solubility Index as defined by the American Oil Chemists' Society Method Ac4-41. "KOH Nitrogen Solubility" is an indicator of soybean meal quality and refers to the amount of nitrogen soluble in 0.036 M KOH under the conditions as described by Araba and Dale [(1990) Poultry Science 69:76-83]. "White" flakes refer to flaked, dehulled cotyledons that have been defatted and treated with controlled moist heat to have an NSI of about 85 to 90. This term can also refer to a flour with a similar NSI that has been ground to pass through a No. 100 U.S. Standard Screen size. "Cooked" refers to a soy protein product, typically a flour, with an NSI of about 20 to 60. "Toasted" refers to a soy protein product, typically a flour, with an NSI below 20.
"Grits" refer to defatted, dehulled cotyledons having a U.S. Standard screen size of between No. 10 and 80.
"Soy Protein Concentrates" refer to those products produced from dehulled, defatted soybeans by three basic processes: acid leaching (at about pH 4.5), extraction with alcohol (about 55 80%), and denaturing the protein with moist heat prior to extraction with water.
Conditions typically used to prepare soy protein concentrates have been described by Pass [(1975) U.S. Pat. No. 3,897,574; Campbell et al., (1985) in New Protein Foods, ed. by Altschul and Wilcke, Academic Press, Vol. 5, Chapter 10, Seed Storage Proteins, pp. 302-338]. "Extrusion" refers to processes whereby material (grits, flour or concentrate) is passed through a jacketed auger using high pressures and temperatures as a means of altering the texture of the material.
"Texturing" and "structuring" refer to extrusion processes used to modify the physical characteristics of the material. The characteristics of these processes, including thermoplastic extrusion, have been described previously [Atkinson, (1970) U.S. Pat. No. 3,488,770, Horan (1985) in New Protein Foods, ed. by Altschul and Wilcke, Academic Press, Vol 1A, Chapter 8, pp 367-414].

Moreover, conditions used during extrusion processing of complex foodstuff mixtures that include soy protein products have been described previously [Rokey (1983) Feed Manufacturing Technology III, 222-237; McCulloch, U.S. Pat. No. 4,454,804].

Seeds from the plants of the present invention express an improved soluble carbohydrate content relative to commercial varieties. The improvements include not only a reduced total raffinose saccharide content, but also a shift from higher (stachyose) to lower (galactinol) molecular weight α-galactosides. The carbohydrate profile of these lines are dramatically different from the profiles seen in elite or germplasm lines used in or produced by other soybean breeding programs.

Applicants teach two separate methods to produce the novel soybean genes of the present invention. The first approach involved exhaustive screening of existing soybean germplasm collections for sources of genes conferring low raffinose saccharide content.
Applicants' germplasm screen was successful despite the failure of previous attempts by others to select and confirm germplasm with significant reduction of raffinose saccharides. The second approach marks the first successful attempt to induce a mutation conferring low raffinose saccharide content. Both approaches resulted in the discovery of soybean genes that can be used to develop soybean lines that are superior (in terms of reduced raffinose saccharide content) to any lines previously reported.

After screening approximately 14,000 lines from germplasm collections, a soybean gene stc1a was discovered in line LR28 and shown to confer a reproducibly low total raffinose saccharide content (Example 1). To confirm its value as a source of altered carbohydrate content, the seed composition of LR28 was compared to that of a number of other PI's and elite lines that have been reported in the literature as having the genetic potential for improving the raffinose saccharide content of soybean. This analysis under identical assay conditions indicated that LR28 displayed a substantially reduced raffinose saccharide content compared to any currently known source of germplasm. Inheritance studies conducted by Applicants indicated that LR28 contains a single recessive to codominant gene (designated stc1a) that confers the low raffinose saccharide trait. Applicants have also demonstrated that high protein content segregates independently of stc1a and that high protein content can be recombined with low raffinose saccharide content by conventional breeding techniques to produce lines having both traits.

The second approach, mutagenesis, resulted in the creation of mutant gene stc1b that confers a low raffinose saccharide phenotype similar to that conferred by stc1a (Example 2). Genetic studies indicated that stc1b is allelic to stc1a. Consequently, it is expected that stc1b can be used as an alternative source of the low raffinose saccharide trait conferred by stc1a. As with stc1a, it is expected that stc1b will be recombined with any other heritable seed trait or agronomic trait of interest. Since the stc1b mutation was induced by Applicants within the genetic background of an elite variety, it is expected that minimal breeding effort will be required to recombine stc1b with desirable agronomic performance. In addition to the major effect of stc1a and stc1b on total raffinose saccharide content, Applicants provide evidence of genetic modifiers that enhance the expression of stc1a (see Example 1).

Careful efforts by Applicants demonstrated conclusively that previous attempts of others to select germplasm with significant reduction in raffinose saccharide content were unsuccessful. Given the perceived value of low raffinose saccharide content, it is surprising that previously reported sources of low raffinose saccharide germplasm have not been used commercially The reason for this discrepancy is the fact that previously identified sources of low raffinose saccharides have been artifacts of environmental variation, poor quality seed, or analytical inconsistencies. Unfortunately, failure of these germplasm sources to confirm have simply not been reported in the literature and may have resulted in wasted effort on the part of soybean breeders who have used such lines for breeding purposes. Prior to Applicants' discovery of stc1a and stc1b, a more critical evaluation of known lines would actually lead one to believe that genetic variation for low raffinose saccharide content does not exist within soybean.
Despite such a forecast, Applicants' exhaustive efforts have resulted in the discovery of truly rare and valuable genes.

If lines containing stc1x are crossed with germplasm sources containing other desirable traits, it is expected that a fraction of the resultant progeny will inherit stc1x in combination with the desirable trait(s) from other said germplasm sources. Desirable seed traits that will be combined with stc1x include (but are not limited to) high protein content, high methionine content, high lysine content, high oleic acid content, high stearic acid content, low palmitic acid content, low linoleic acid content, low linolenic acid content, lipoxygenase nulls, and trypsin inhibitor nulls. It is also expected that stc1x will be combined with any trait of argonomic significance to develop elite lines. Examples of such agronomic traits include (but are not limited to) emergence vigor, seedling vigor, vegetative vigor, disease resistance, pest resistance, herbicide resistance, drought resistance, lodging resistance, and high seed yield.

To demonstrate the effect of stc1x on the nutritional quality of soybeans, defatted, toasted soybean meals were prepared from lines homozygous for stc1x (low in raffinose saccharide content) and from conventional soybean lines (with normal raffinose saccharide content). The meals were assayed to determine their nitrogen-corrected, True Metabolizable Energy (TME_{N}) content for broilers. Meals from stc1x lines had significantly higher (ca. 12%) TME_{N} and greater utilization of gross energy compared to meals from the conventional soybean lines. Even relatively modest increases in metabolizable energy content of a major feedstuff such a soybean meal can have major economic benefits for the animal feed and animal production industries, due to the extraordinarily large flocks that most commercial opertations maintain. The improvement in quality of soybean meal from stc1x lines should provide an excellent opportunity to further increase the efficiency of animal husbandry throughout the world.

The utility of stc1x lines was further demonstrated by preparing pet foods from defatted, toasted meals from stc1a lines and comparing their raffinose saccharide composition to pet foods produced from meals from conventional soybean lines. Lines homozygous for stc1a produced soybean meal and a food with a substantially lower raffinose saccharide content than those from conventional lines. U.S. Patent 4,454,804 (McCulloch), incorporated by reference herein, discloses methods for the production of such a pet food product. The product typically includes farinaceous ingredients such as wheat, corn, barley, oats, and the like, or their derivatives such as corn meal, hominy, wheat middlings, wheat germ, etc. Typically, the amount of farinaceous ingredients in the expandable mixture comprises between about 30% to 70% by weight of the mixture.

The product may also include one or more proteinaceous ingredients of vegetable, animal or fish origin such as soybean meal, soy grits, meat meal, bone meal, poultry meal, fish scrap and combinations thereof. Typically, the proteinaceous ingredients comprise between about 20% and 50% by weight of the mixture.

The balance of the mixture may comprise salts, flavorings, colorings, vitamin supplements, minerals and other like ingredients to form a nutritionally balanced pet food product.

The extrusion zone of production of the pet food product experiences temperatures substantially above 212°F, and preferably between 250°F and 350°F The pressures developed within the extruder at the die plate should be above the vapor pressure of water at its extrusion temperature, typically between about 25-600 psi.

In addition, defatted, white flakes were prepared from stc1a lines and conventional lines and their raffinose saccharide contents compared. White flakes from stc1a lines displayed a substantially reduced raffinose saccharide content compared to those from conventional lines. In addition, white flakes from stc1a lines displayed a substantially lower raffinose saccharide content than several commercially obtained soy protein products (flours, textured flour, concentrate, textured concentrate). This improved composition should enable manufacturers of these products to obtain improved quality of their final product. They should achieve the added benefit of increased efficiency in their manufacturing processes as considerable resources are needed to build and operate processing plants that have been designed to reduce the raffinose saccharide content during the manufacture of soy protein concentrates and isolates.

As was seen with the other commercial soy products, the white flakes from stc1a lines have a substantially improved raffinose saccharide content compared to that in the commercial soyfood products. Soyfood manufacturers could achieve many of the same benefits of improved nutritional quality and processing efficiency with the manufacture of other soy protein products described above.

The Applicants emphasize that the conditions described above were chosen to be representative to those found in commercial operations for the production of a desolventized, toasted high (i.e., >47.5% at 12% moisture) protein meal. The precise conditions used to process the materials will undoubtedly vary from those described in the instant invention, depending on the specific manufacturing process. However, by using conventional methods [see JAOCS (1981) 11, Number 3] to crack, dehull, flake, extract, and desolventize/toast, commercial operations should be able to prepare soy protein products from stc1x lines as easily as they currently do with conventional soybeans. Moreover, soy protein product manufacturers could choose to prepare other soy protein products by employing alternate processing conditions. For example, products with higher oil contents could be produced by not employing solvent extraction (e.g., full-fat products). In addition, alternative solvent extraction conditions (e.g., supercritical gas such a carbon dioxide) or solvents other than hexane (e.g., alcohols, methyl ethyl ketone, acetone, etc.) could be employed. Lower (e.g., minimum 44% protein at 12% moisture) protein products could be produced by altering the conditions used to separate hulls from the cracked meats or by adding hulls back to more highly processed materials. Further, edible products such as soyflours could be produced by using alternate toasting conditions (e.g., flash desolventization). Finally, the properties of soy protein products from stc1x lines could be affected through the use of processes such as, but not limited to, extrusion, jet cooking, or homogenization.
Collectively, the results clearly demonstrate that the soybean genes discovered by Applicants have widespread utility for the production of improved soy protein products from soybeans with inherently low raffinose saccharide content.

### EXAMPLES

The present invention is further defined in the following Examples, in which all parts and percentages are by weight and degrees are Celsius, unless otherwise stated. It should be understood that these Examples, while indicating preferred embodiments of the invention, are given by way of illustration only. Examples in which the stachyose content is not less than 30 µmol/g (based on undried seed) do not fall within the scope of the claims.

### EXAMPLE 1

### A SOYBEAN GENE, stc1a, CONFERRING IMPROVED CARBOHYDRATE COMPOSITION

### Assays for Raffinose Saccharides and Soluble Carbohydrates

Raffinose saccharide content was determined using two distinct assays. Typically 5 to 10 soybeans from a given plant were ground in a Cyclotech 1093 Sample Mill (Tecator, Box 70 S-26301, Hoganas, Sweden) equipped with a 100 mesh screen. Except where noted, total α-galactoside or raffinose saccharide content were determined on an "as is" basis for the powders. For comparison among certain lines or among certain soy protein products, the ground seed powder was placed in a forced air oven at 45°C until the samples reached constant weight. All tables herein will list whether carbohydrate content is expressed on an "as is" or dry matter ("db") basis. Moisture content ranged from 6 to 13% in products characterized on an "as is" basis. Approximately 30 mg of the resultant powder were weighed into a 13 x 100 mm screw cap tube and 1.6 mL of chloroform and 1.4 mL of methanol:water (4:3, v/v) was added. The precise powder weight of each sample was recorded and used to adjust the following assay results for sample to sample weight differences. The tubes were then capped, placed in racks and shaken on a rotary shaker for 60 min at 1800 rpm at room temperature.
After extraction, the contents of the tubes were allowed to settle for 15 min. After settling, a 15 µL aliquot of the methanol:water phase was placed in a well of a 96 well microtiter plate and dried at 45°C for 20 min. At this point the raffinose saccharide content was determined in one of two assays. The first involved a coupled enzymatic assay that employs α-galactosidase and galactose dehydrogenase as described previously (Schiweck and Busching (1969) Zucker 22:377-384, Schiweck and Busching (1975) Zucker 28:242-243, Raffinose Detection Kit^{®}, Boehringer Mannheim GMBH, Catalog Number 428 167) with modifications of the assay conditions. The modifications of the assay included addition of Bovine Serum Albumin (15 mg/mL) to the assay and α-galactosidase buffers, increasing the temperature and time of the α-galactosidase incubation from room temperature to 45°C and 30 min, and increasing the time of the galactose dehydrogenase incubation from 20 min to 60 min and using stachyose instead of raffinose for the α-galactoside standard. After incubation, the A₃₄₀ of the samples were determined on a BIO-TEK^{®} Model EL340 Microplate reader. The amount of α-galatosides present in the samples were determined by comparison to known quantities of the stachyose standard.

To facilitate the analysis of thousands of samples, initial assays were replicated once. Lines that appeared to be low in raffinose saccharide content from this primary assay were subsequently reassayed in triplicate, beginning from the ground seed, if sufficient material was available. Lines whose composition was confirmed in the secondary assay were grown to maturity under field conditions and seed from the field grown plants were assayed again. Lines that displayed a reduced raffinose saccharide phenotype using the coupled enzymatic assay after being grown in the field were then reassayed using the following HPAEC/PAD raffinose saccharide assay. The additional assay was used to eliminate the potential for artifacts that could result from the use of an enzymatically based assay (e.g., the presence of a novel inhibitor of α-galactosidase or galactose dehydrogenase in the seed), as well as to obtain more complete information of the individual soluble carbohydrates present in the seed.

A High Performance Anion Exchange Chromatography/Pulsed Amperometric (HPAEC/PAD) assay was used for determining the content of individual raffinose saccharides (e.g., stachyose, raffinose, and galactinol). Conditions for the grinding and extraction of the seed were identical to those used for the previous assay. A 750 µL aliquot of the aqueous phase was removed and dried under reduced pressure at 80°C. The dried material was then dissolved in 2 mL of water, mixed vigorously for 30 sec. A 100 µL aliquot was removed and diluted to 1 mL with water. The sample was mixed thoroughly again and then centrifuged for 3 min at 10,000 x g. Following centrifugation, a 20 µL sample was analyzed on a Dionex™ PA1 column using 150 mM NaOH at 1.3 mL/min at room temperature. The Dionex™ PAD detector was used with E₁=0.05 v, E₂=0.60 v and E₃= -0.60 v and an output range of 3 µA. Galactinol, glucose, fructose, sucrose, raffinose, stachyose and verbascose were well separated by the chromatographic conditions. The carbohydrate content of the samples was determined by comparison to authentic standards. Total raffinose saccharide content was determined by the sum of the galactinol, raffinose and two times the stachyose content of the respective samples. This value is a reflection of the total number of α-linked galactose residues present in these carbohydrates as measured by the HPAEC/PAD method described above.

Results obtained from the carbohydrate analyses were subjected to analysis of variance using the software SuperANOVA (Abacus Concepts, Inc., 1984 Bonita Avenue, Berkeley, CA 94704). When appropriate, Fisher's Protected LSD was used as the post-hoc test for comparison of means. In other comparisons, means were considered statistically significant if the ranges defined by their standard errors (SEM's) did not overlap.

### Near Infrared Transmittance (NIT) Assay for Seed Protein Content

Seed protein content was determined nondestructively by Near Infrared Transmittance (NIT) using a Tecator™ Model 1255 Food and Feed Analyzer (Tecator AB, Box 70, S-263 21, Hoganas Sweden). The protein values used for the calibration equation were determined using the Kjeldahl digestion method (JAOAC (1976) 59:141). The calibration set included 75 soybean samples that ranged from to to 50.9% protein on a dry matter basis (DB). Infratec Calibration Maker software (Infra-Maker: Produced for Tecator AB by Camo, Norway) was used according to the manufacturer's protocol in the development of the calibration equation. Approximately 10 g of seed per line were used in the analysis.

### Screening Soybean Germplasm for Improved Carbohydrate Composition

Using the above carbohydrate assays, a total of ca. 14,000 PI lines from the USDA Soybean Germplasm Collection were assayed for total α-galactoside content and/or total raffinose saccharides content. After primary and secondary assays of PI lines, 25 lines (Table 2) were grown in the field to determine whether the low raffinose saccharide phenotype was heritable (expressed in subsequent generations). Of the original 25 PI selections that appeared to be low in raffinose saccharide phenotype, only the phenotype of LR28 was heritable. LR28 displayed the lowest total α-galactoside content after being grown again under field conditions (Table 2). Presumably, the low raffinose saccharide phenotypes of the other 24 PI selections were artifacts created by the age and storage conditions of the seeds assayed. For example, some of the candidates from the initial assays, particularly PI 416.815 (LR1) and PI 408.277 (LR2), had poor seed quality and poor germination ability compared to the other candidates. The low raffinose saccharide content of LR1 and LR2 in the initial screen was possibly due to the fact that the seed obtained from the UDSA for screening was old and had metabolized most of its carbohydrate reserves. Such selection artifacts typify the obstacles associated with the selection of lines that have genetic variation for low raffinose saccharide content. It is therefore essential that any germplasm source be regrown and reassayed before it can be confirmed as a heritable source of low raffinose saccharide content. Applicants not only have confirmed the heritibility of the germplasm sources disclosed in the current application, but also have characterized the inheritance of the low raffinose saccharide content trait. Prior reports by others of low raffinose saccharide content have not been substantiated with such rigorous confirmation.

**TABLE 2**

| Confirmation of LA28 as a Germplasm Source for Low Seed Soluble α-Galactoside Content | | | |
|---|---|---|---|
| Low Raffinose Saccharide Candidate | PI Identification Number | Secondary Screen Total Galactoside µmoles/g (as is) | Grown Again in Field Total α-Galactoside µmoles/g (as is) |
| LR1 | PI 416.815 | 64.9 | 146.2 |
| LR2 | PI 408.277 | 88.1 | 158.7 |
| LR3 | PI 408.31 0A | 102.5 | 145.8 |
| LR4 | PI 423.753A | 102.2 | 148.8 |
| LR5 | PI 408.123 | 105.4 | 146.1 |
| LR6 | PI 398.649 | 111.7 | 145.0 |
| LR7 | PI 408.105A | 111.5 | 170.6 |
| LR8 | PI 1416.923 | 118.0 | 175.7 |
| LR9 | PI 404.159 | 125.2 | 179.9 |
| LR10 | PI 398.965 | 129.0 | 167.1 |
| LR11 | PI399.073 | 133.1 | 144.1 |
| LR17 | PI407.805A | 118.6 | 155.6 |
| LR18 | PI 407.888 | 112.2 | 155.2 |
| LR19 | PI 399.089 | 114.9 | 150.1 |
| LR20 | PI 407.921 | 128.4 | 170.6 |
| LB21 | PI 408.140B | 128.5 | 160.0 |
| LR24 | PI 227.558 | 124.3 | 191.6 |
| LR27 | PI 157.490 | 111.4 | 160.7 |
| LR28 | PI 200.508 | 86.0 | 107.7 |
| LR29 | PI 157.459 | 130.3 | 149.6 |
| LR30 | PI 248.512 | 107.3 | 193.2 |
| LR31 | PI 253.653 | 119.1 | 149.6 |
| LR32 | PI 290.114 | 122.1 | 176.4 |
| LR33 | PI OZZIE | 122.1 | 173.0 |
| LR34 | PI HAROSOY | 114.3 | 161.8 |

### Superiority of LR28 to Previously Reported Germplasm

To investigate the novelty of the raffinose saccharide content of LR28, it was compared under identical analytical conditions to a series of elite check lines and to a series of PI lines that had been previously reported as being low in raffinose saccharide content [see Hymowitz, et al. (1972) Comm. In Soil Science and Plant Analysis 3:367-373, Hymowitz et al. (1972) Agronomy J. 64:613-616, Hymowitz and Collins (1974) Agronomy J. 66:239-240, Openshaw and Hadley (1978) Crop Science 18:581-584, Openshaw and Hadley (1981) Crop Science 21:805-808, and Saravitz (1986) Ph.D. Thesis, North Carolina State University, Horticultural Science Department].

Seeds of LR28, 8 elite check lines (A3205, Acme, Ajma, Altona, Bonus, Fiskeby, Norman, and Portage), and said previously reported PI's were assayed by the HPAEC/PAD method for total raffinose saccharide content (Table 3). LR28 was substantially lower in total raffinose saccharide content than all elites lines and said previously reported lines of relevance that were tested. All but one of the previously reported PI lines fell within or above the range of total raffinose saccharide content of the elite check lines. The best of the previously reported PI's (P1203.246) was only 3.3 µmol/g lower in total raffinose saccharide content than the lowest of the elite check lines. This is clearly inferior to LR28 which was 47.3 µmol/g lower than the best elite check line. In fact, most of the previously reported PI lines were actually higher in raffinose saccharide content than the range defined by the elite checks. This demonstrates the lack of repeatability of prior attempts to identify low raffinose saccharide germplasm.

When the stachyose content of LR28 is compared to that of the other lines (Table 3), it is quite convincing that LR28 is a truly unique germplasm line. The stachyose content of LR28 was 55% lower than the next lowest line (PI203.246) and approximately 65% lower than the average stachyose content of the elite check lines. This is relevant since stachyose is the most abundant of the raffinose saccharides present in soybeans and is thought to be the most undesirable from a nutritional standpoint [Cristofaro et al. (1974) in Sugars in Nutrition, Ch 20, 313-335].

**TABLE 3**

| Comparison of the Carbohydrate Composition of LR28 With Elite Check Lines and PI's That Have Been Suggested as Germplasm With Low Raffinose Saccharide Content | | | | |
|---|---|---|---|---|
| | Total Raffinose Saccharide | Stachyose | Raffinose | Galactinol |
| Line | µmoles/g dry basis | µmoles/g dry basis | µmoles/g dry basis | µmoles/g dry basis |
| LR28 | 114.7 | 27.9 | 7.8 | 51.1 |
| A3205 | 162.0 | 72.5 | 17.1 | 0.0 |
| ACME | 182.5 | 76.5 | 29.6 | 0.0 |
| AJMA | 184.7 | 80.2 | 24.3 | 0.0 |
| ALTONA | 181.9 | 79.6 | 22.8 | 0.0 |
| BONUS | 182.5 | 81.4 | 19.8 | 0.0 |
| FISKEBY | 192.0 | 85.8 | 20.4 | 0.0 |
| NORMAN | 185.4 | 80.5 | 24.4 | 0.0 |
| PORTAGE | 193.0 | 84.9 | 23.2 | 0.0 |
| PI79.593 | 211.3 | 89.3 | 24.4 | 8.3 |
| PI79.727 | 176.5 | 80.3 | 13.2 | 2.7 |
| PI80.488-1 | 205.7 | 90.1 | 19.8 | 5.7 |
| PI81.761MD | 201.0 | 89.0 | 23.0 | 0.0 |
| PI81.761YD | 186.3 | 80.5 | 25.4 | 0.0 |
| PI81.763 | 195.1 | 86.5 | 19.2 | 2.9 |
| PI81.766 | 228.2 | 100.9 | 19.6 | 6.9 |
| PI81.768 | 201.5 | 91.9 | 17.6 | 0.0 |
| PI81.770 | 205.9 | 93.8 | 15.9 | 2.6 |
| PI81.771 | 204.0 | 92.4 | 17.7 | 1.4 |
| PI81.772 | 193.9 | 88.1 | 13.5 | 4.2 |
| PI81.773 | 231.5 | 104.0 | 20.0 | 3.4 |
| PI81.785 | 237.0 | 101.7 | 25.4 | 8.2 |
| PI86.002 | 271.8 | 120.5 | 24.7 | 6.1 |
| PI86.046 | 246.5 | 109.2 | 22.8 | 5.3 |
| PI135.624 | 211.8 | 91.2 | 23.8 | 5.6 |
| PI153.292 | 213.3 | 94.0 | 24.6 | 0.8 |
| PI163.453 | 217.0 | 95.9 | 25.3 | 0.0 |
| PI189.950 | 238.4 | 108.6 | 16.7 | 4.5 |
| PI203.246 | 158.7 | 61.7 | 16.9 | 27.7 |
| PI232.987 | 249.2 | 112.4 | 23.1 | 1.4 |
| PI232.989 | 253.8 | 116.3 | 21.2 | 0.0 |
| PI232.991 | 299.1 | 134.1 | 26.2 | 4.7 |
| PI326.581 | 197.8 | 86.1 | 16.1 | 9.5 |
| PI339.731 | 177.9 | 77.1 | 20.8 | 2.9 |
| PI342.434 | 184.0 | 82.9 | 18.2 | 0.0 |
| PI361.123A | 188.4 | 82.3 | 23.8 | 0.0 |
| PI361.123B | 182.6 | 80.5 | 21.5 | 0.0 |

### Inheritance of the Improved Carbohydrate Composition of LR28

To study the inheritance of the low raffinose saccharide phenotype of LR28, the line was crossed with four different elite lines. The four elite lines used were A4715 (Asgrow Seed Co. variety), X3337 (Asgrow Seed Co. elite), ST9025 (E. I. du Pont de Nemours and Company, elite), and ST9026 (E. I. du Pont de Nemours and Company, elite). F1 seeds were grown in the greenhouse and allowed to self-pollinate. The resulting F2 seeds were then planted in the field and the resultant F1 plants allowed to self-pollinate. F3 seeds derived from individual F2 plants (F2 lines) were assayed for total α-galactoside content using the method described in Example 1. Total α-galactoside content was used to score F2 lines for Mendelian genetic segregation studies.

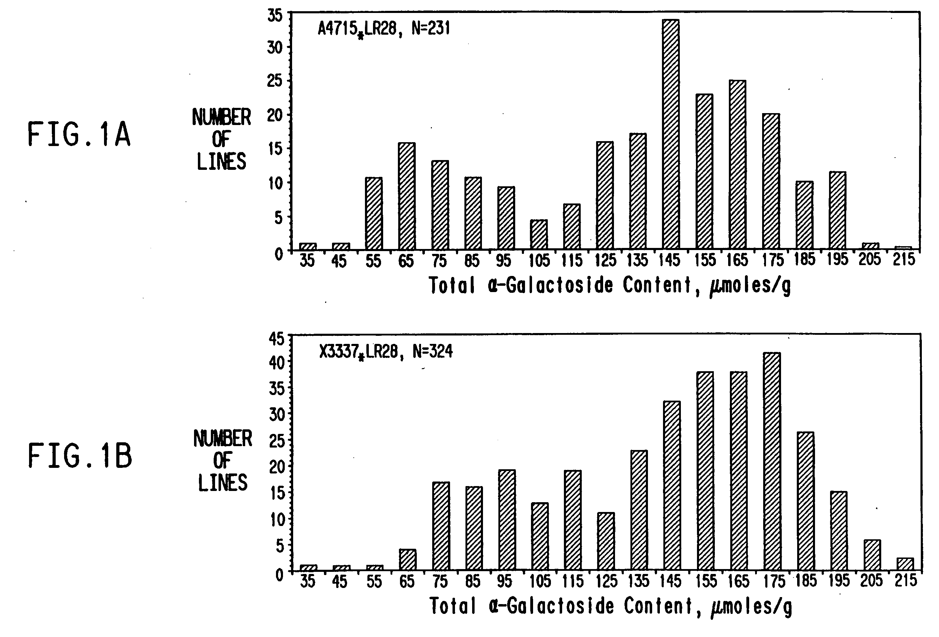

Segregation for total α-galactoside content in all four elfte*LR28 crosses (Figures 1 A-D) followed a bimodal distribution in which the F2 lines could be grouped into one of two discrete classes: those with seed containing less than 110 µmol/g of total α-galactoside ("low" class) and those with seed containing more than 110 µmol/g total α-galactoside (the "high" class). The "low" class covered the range of α-galactoside levels normally observed among single plants of parent LR28. The "high" class included the range of α-galactoside contents previously observed among single plants of conventional lines and also included the intermediate range of α-galactoside levels that would be expected for lines derived from F2 plants that were heterozygous for any gene(s) conferring low raffinose saccharide content. In all four F2 populations, the ratio of "low" to "high" F2 lines was not significantly different than a ratio of 1 to 3 when subjected to χ² analysis (Table 4). Since the range of intermediate phenotypes indicative of heterozygous plants was continuous with the remainder of the "high" class, low raffinose saccharide content is either recessive or codominant in terms of gene action. Segregation between the high and low class was consistent with segregation of two alleles at a single locus where the allele from the conventional lines, herein named "Stc1" (after stachyose), confers high (conventional) raffinose saccharide content and the allele from LR28, herein named "stc1a", confers the low raffinose saccharide phenotype. Results indicate that stc1a must be in the homozygous condition for full expression of the low raffinose saccharide phenotype. The fact that the stc1a homozygotes can be distinguished from heterozygotes (as indicated by a relatively quick enzymatic assay) is of particular importance for breeding applications. This ensures that lines selected below an appropriately low raffinose saccharide threshold will be fixed (in the homozygous condition) for the stc1a allele. Due to the indication that intermediate phenotypes represent heterozygous individuals, it should also be possible to predictably select heterozygous individuals when necessary in certain breeding applications. The predictable inheritance of stc1a will greatly facilitate its transfer into elite lines through conventional breeding techniques. It is believed that the gene will be useful in any genetic background for improving the carbohydrate composition of the seed.

**TABLE 4**

| Segregation for Raffinose Saccharide Content in Four Elite*LR28 F2 Populations | | | | |
|---|---|---|---|---|
| Cross | F2 Segregation Class | | χ² for 1:3 Ratio | Statistical Significance |
| | "low" | "high" | | |
| A4715*LR28 | 66 | 165 | 1.57 | ns |
| X3337*LR28 | 72 | 252 | 1.33 | ns |
| ST9025*LR28 | 59 | 182 | 0.03 | ns |
| ST9026*LR28 | 65 | 218 | 0.62 | ns |

### Evidence for Genetic Modifiers of stc1a a Within Elite Lines

Using seed from the four previously described elite*LR28 populations, total raffinose saccharide content was measured on F2 lines with a total α-galactoside content less than 110 µmoles/g (as is) using the HPAEC/PAD method described in Example 1. Based on the inheritance study described above, these lines represent the class of segregants that are homozygous for the stc1a allele from parent LR28. For lines with adequate seed, protein content was also measured using NIR reflectance as described in Example 1. Stachyose, raffinose, galactinol, total raffinose saccharide, and protein content of lines with less than 110 µmoles/g (as is) total raffinose saccharide content are shown in Table 5.

**TABLE 5**

| Seed Components of F2 Lines Homozygous for the stc1a Allele From Elite*LR28 Crosses Sorted by Elite Parent and Stachyose Content | | | | | | |
|---|---|---|---|---|---|---|
| Pedigree | F2 Line | STC | RAF | GAL | RSAC | PRO |
| | | -- µmoles/g, as is -- | | | | %, db |
| A4715*LR28 | 6.126 | 11.3 | 5.7 | 42.4 | 70.7 | 42.7 |
| A4715*LR28 | 6.168 | 12.1 | 6.1 | 26.3 | 56.6 | -- |
| A4715*LR28 | 6.066 | 12.3 | 5.2 | 32.3 | 62.0 | -- |
| A4715*LR28 | 6.163 | 13.1 | 6.1 | 34.6 | 66.9 | -- |
| A4715*LR28 | 6.012 | 13.2 | 5.4 | 51.4 | 83.1 | 42.0 |
| A4715*LR28 | 6.224 | 15.5 | 6.1 | 35.0 | 72.0 | 46.2 |
| A4715*LR28 | 6.088 | 15.7 | 6.7 | 49.3 | 87.4 | -- |
| A4715*LR28 | 6.249 | 16.2 | 5.8 | 27.7 | 65.8 | -- |
| A4715*LR28 | 6.159 | 17.5 | 6.8 | 43.2 | 85.1 | -- |
| A4715*LR28 | 6.318 | 17.6 | 7.5 | 37.2 | 79.8 | -- |
| A4715*LR28 | 6.111 | 18.7 | 7.5 | 45.4 | 90.3 | 42.7 |
| A4715*LR28 | 6.183 | 19.5 | 7.5 | 33.2 | 79.7 | 43.3 |
| A4715*LR28 | 6.057 | 19.5 | 8.3 | 34.4 | 81.7 | 45.6 |
| A4715*LR28 | 6.125 | 19.7 | 6.9 | 40.2 | 86.5 | 43.0 |
| A4715*LR28 | 6.187 | 19.8 | 7.4 | 32.0 | 79.0 | 44.8 |
| A4715*LR28 | 6.103 | 20.3 | 7.4 | 44.5 | 92.5 | 43.8 |
| A4715*LR28 | 6.196 | 20.4 | 7.2 | 39.0 | 87.0 | 45.1 |
| A4715*LR28 | 6.162 | 20.9 | 8.0 | 29.4 | 79.2 | -- |
| A4715*LR28 | 6.073 | 21.0 | 8.7 | 35.7 | 86.4 | 45.3 |
| A4715*LR28 | 6.122 | 21.6 | 7.9 | 28.5 | 79.6 | 44.1 |
| A4715*LR28 | 6.068 | 21.7 | 8.0 | 30.7 | 82.2 | -- |
| A4715*LR28 | 6.213 | 21.8 | 7.5 | 28.1 | 79.3 | 43.9 |
| A4715*LR28 | 6.010 | 22.0 | 144.8 | 32.5 | 91.2 | 47.0 |
| A4715*LR28 | 6.044 | 22.5 | 8.1 | 28.8 | 81.9 | 44.6 |
| A4715*LR28 | 6.083 | 22.8 | 8.4 | 34.5 | 88.6 | 43.5 |
| A4715*LR28 | 6.107 | 23.0 | 8.2 | 31.0 | 85.3 | 44.9 |
| A4715*LR28 | 6.039 | 23.6 | 9.3 | 28.1 | 84.7 | 45.6 |
| A4715*LR28 | 6.184 | 24.1 | 7.8 | 26.7 | 82.7 | 43.5 |
| A4715*LR28 | 6.097 | 24.2 | 8.7 | 35.2 | 92.4 | -- |
| A4715*LR28 | 6.084 | 24.6 | 8.5 | 40.8 | 98.5 | -- |
| A4715*LR28 | 6.033 | 24.7 | 8.6 | 35.6 | 93.7 | 43.7 |
| A4715*LR28 | 6.123 | 25.3 | 9.0 | 27.3 | 86.9 | 48.1 |
| A4715*LR28 | 6.135 | 25.8 | 8.5 | 28.3 | 88.3 | -- |
| A4715*LR28 | 6.277 | 26.0 | 8.1 | 37.4 | 97.5 | 44.1 |
| A4715*LR28 | 6.143 | 26.3 | 9.1 | 42.5 | 104.1 | 44.1 |
| A4715*LR28 | 6.055 | 27.7 | 9.3 | 22.6 | 87.3 | 46.3 |
| A4715*LR28 | 6.004 | 28.2 | 8.9 | 30.9 | 96.1 | 43.3 |
| A4715*LR28 | 6.120 | 29.1 | 9.8 | 29.8 | 97.9 | 42.6 |
| A4715*LR28 | 6.221 | 29.4 | 8.7 | 30.6 | 98.1 | -- |
| A4715*LR28 | 6.121 | 29.8 | 9.9 | 29.7 | 99.3 | 43.3 |
| A4715*LR28 | 6.056 | 30.1 | 8.5 | 27.5 | 96.2 | 43.9 |
| A4715*LR28 | 6.206 | 30.2 | 9.2 | 22.3 | 92.0 | 45.6 |
| A4715*LR28 | 6.102 | 32.2 | 10.1 | 31.8 | 106.2 | 46.8 |
| A4715*LR28 | 6.208 | 34.8 | 9.5 | 25.6 | 104.7 | 43.7 |
| A4715*LR28 | 6.194 | 38.5 | 10.5 | 19.2 | 106.7 | 43.8 |
| X3337*LR28 | 5.364 | 12.9 | 7.9 | 40.6 | 74.4 | 47.4 |
| X3337*LR28 | 5.279 | 13.4 | 6.7 | 45.0 | 78.4 | 42.8 |
| X3337*LR28 | 5.013 | 13.5 | 6.3 | 20.3 | 53.6 | 45.9 |
| X3337*LR28 | 5.277 | 15.3 | 6.9 | 40.5 | 78.0 | 44.1 |
| X3337*LR28 | 5.171 | 16.3 | 6.5 | 33.5 | 72.5 | 44.2 |
| X3337*LR28 | 5.356 | 16.4 | 6.5 | 37.0 | 76.3 | 43.5 |
| X3337*LR28 | 5.153 | 17.7 | 7.3 | 33.6 | 76.2 | 42.2 |
| X3337*LR28 | 5.365 | 17.9 | 6.3 | 34.5 | 76.5 | 45.3 |
| X3337*LR28 | 5.299 | 18.0 | 7.5 | 30.7 | 74.1 | -- |
| X3337*LR28 | 5.318 | 18.4 | 7.6 | 32.6 | 76.9 | 43.8 |
| X3337*LR28 | 5.276 | 19.0 | 8.5 | 27.0 | 73.5 | 42.2 |
| X3337*LR28 | 5.298 | 19.2 | 7.9 | 32.6 | 78.7 | 45.5 |
| X3337*LR28 | 5.152 | 19.7 | 7.5 | 28.3 | 75.2 | 43.5 |
| X3337*LR28 | 5.111 | 19.7 | 7.8 | 28.0 | 75.3 | 42.4 |
| X3337*LR28 | 5.194 | 20.1 | 8.3 | 36.4 | 84.9 | 43.5 |
| X3337*LR28 | 5.147 | 20.2 | 8.0 | 28.0 | 76.3 | 42.6 |
| X3337*LR28 | 5.207 | 20.5 | 8.3 | 25.1 | 74.4 | 43.0 |
| X3337*LR28 | 5.150 | 20.5 | 7.9 | 28.1 | 77.0 | 43.7 |
| X3337*LR28 | 5.215 | 20.5 | 7.8 | 30.5 | 79.4 | 44.2 |
| X3337*LR28 | 5.170 | 20.5 | 7.9 | 37.1 | 86.1 | 44.4 |
| X3337*LR28 | 5.162 | 20.6 | 7.8 | 23.2 | 72.1 | 44.2 |
| X3337*LR28 | 5.037 | 20.6 | 8.6 | 35.6 | 85.4 | 44.9 |
| X3337*LR28 | 5.116 | 21.8 | 8.1 | 32.8 | 84.5 | -- |
| X3337*LR28 | 5.103 | 21.9 | 8.7 | 22.1 | 74.5 | 45.6 |
| X3337*LR28 | 5.142 | 22.1 | 7.7 | 19.9 | 71.9 | -- |
| X3337*LR28 | 5.220 | 22.9 | 8.2 | 31.3 | 85.3 | 42.4 |
| X3337*LR28 | 5.268 | 23.2 | 7.7 | 26.9 | 81.1 | 43.7 |
| X3337*LR28 | 5.093 | 23.2 | 10.0 | 25.1 | 81.4 | 43.1 |
| X3337*LR28 | 5.044 | 23.8 | 8.7 | 32.8 | 89.1 | 41.3 |
| X3337*LR28 | 5.297 | 24.2 | 9.9 | 27.9 | 86.2 | 43.8 |
| X3337*LR28 | 5.074 | 24.2 | 9.0 | 30.7 | 88.2 | 41.5 |
| X3337*LR28 | 5.071 | 24.4 | 8.7 | 35.3 | 92.8 | 42.3 |
| X3337*LR28 | 5.098 | 24.6 | 9.1 | 39.7 | 97.9 | 44.6 |
| X3337*LR28 | 5.131 | 24.7 | 9.2 | 30.9 | 89.5 | -- |
| X3337*LR28 | 5.340 | 24.9 | 9.8 | 27.5 | 87.1 | 43.3 |
| X3337*LR28 | 5.238 | 25.0 | 8.5 | 27.1 | 85.5 | 45.3 |
| X3337*LR28 | 5.330 | 25.1 | 8.5 | 23.9 | 82.5 | 43.5 |
| X3337*LR28 | 5.091 | 25.5 | 9.2 | 38.5 | 98.8 | 41.7 |
| X3337*LR28 | 5.274 | 25.7 | 8.8 | 16.8 | 77.0 | 44.2 |
| X3337*LR28 | 5.110 | 26.1 | 9.5 | 28.3 | 90.0 | 44.1 |
| X3337*LR28 | 5.267 | 26.3 | 9.9 | 26.6 | 89.1 | -- |
| X3337*LR28 | 5.020 | 26.4 | 8.2 | 22.2 | 83.3 | 44.5 |
| X3337*LR28 | 5.229 | 26.7 | 9.4 | 24.0 | 86.8 | 43.1 |
| X3337*LR28 | 5.339 | 26.8 | 10.3 | 26.9 | 90.6 | 42.8 |
| X3337*LR28 | 5.206 | 27.2 | 9.1 | 23.5 | 86.9 | 42.7 |
| X3337*LR28 | 5.134 | 27.4 | 9.5 | 23.0 | 87.2 | -- |
| X3337*LR28 | 5.243 | 28.4 | 10.0 | 19.5 | 86.2 | 43.8 |
| X3337*LR28 | 5.115 | 28.4 | 9.2 | 21.0 | 87.1 | -- |
| X3337*LR28 | 5.219 | 28.9 | 9.6 | 20.2 | 87.7 | -- |
| X3337*LR28 | 5.137 | 29.2 | 10.2 | 19.1 | 87.7 | -- |
| X3337*LR28 | 5.344 | 30.0 | 10.8 | 22.2 | 93.1 | 44.9 |
| X3337*LR28 | 5.263 | 31.0 | 10.1 | 18.7 | 90.0 | 42.7 |
| X3337*LR28 | 5.050 | 32.2 | 10.3 | 24.4 | 99.0 | 41.7 |
| X3337*LR28 | 5.348 | 33.7 | 11.4 | 25.7 | 104.5 | 43.7 |
| ST9025*LR28 | 3.047 | 18.3 | 6.1 | 27.5 | 70.2 | 43.4 |
| ST9025*LR28 | 3.234 | 18.8 | 7.5 | 24.7 | 69.8 | 45.6 |
| ST9025*LR28 | 3.218 | 19.0 | 7.7 | 22.2 | 68.0 | 46.4 |
| ST9025*LR28 | 3.273 | 19.9 | 7.2 | 28.2 | 75.3 | 43.9 |
| ST9025*LR28 | 3.232 | 20.4 | 6.8 | 27.9 | 75.5 | -- |
| ST9025*LR28 | 3.235 | 20.7 | 7.9 | 25.9 | 75.2 | 45.6 |
| ST9025*LR28 | 3.261 | 21.8 | 8.0 | 29.0 | 80.7 | 47.4 |
| ST9025*LR28 | 3.115 | 22.7 | 8.5 | 18.0 | 71.9 | 43.3 |
| ST9025*LR28 | 3.190 | 22.9 | 7.8 | 23.7 | 77.3 | 45.3 |
| ST9025*LR28 | 3.241 | 23.1 | 9.1 | 20.8 | 76.1 | 45.2 |
| ST9025*LR28 | 3.022 | 23.3 | 7.5 | 17.9 | 71.9 | 45.5 |
| ST9025*LR28 | 3.036 | 23.6 | 6.6 | 21.9 | 75.7 | 43.7 |
| ST9025*LR28 | 3.039 | 24.0 | 7.3 | 23.2 | 78.4 | 44.9 |
| ST9025*LR28 | 3.028 | 24.1 | 7.2 | 15.1 | 70.5 | 45.3 |
| ST9025*LR28 | 3.188 | 24.3 | 8.6 | 23.1 | 80.3 | 44.8 |
| ST9025*LR28 | 3.225 | 24.4 | 8.5 | 18.0 | 75.4 | -- |
| ST9025*LR28 | 3.198 | 24.7 | 7.6 | 17.6 | 74.5 | 44.6 |
| ST9025*LR28 | 3.233 | 26.1 | 9.0 | 14.8 | 76.0 | 44.9 |
| ST9025*LR28 | 3.101 | 26.4 | 9.2 | 21.2 | 83.2 | 45.2 |
| ST9025*LR28 | 3.059 | 26.6 | 8.1 | 30.4 | 91.9 | 44.5 |
| ST9025*LR28 | 3.228 | 26.8 | 8.8 | 27.2 | 89.6 | 46.2 |
| ST9025*LR28 | 3.290 | 27.4 | 8.1 | 27.8 | 90.8 | 44.6 |
| ST9025*LR28 | 3.259 | 27.6 | 9.3 | 20.4 | 85.0 | -- |
| ST9025*LR28 | 3.219 | 28.0 | 9.6 | 19.0 | 84.6 | 46.3 |
| ST9025*LR28 | 3.109 | 29.3 | 9.1 | 21.8 | 89.6 | 45.3 |
| ST9025*LR28 | 3.107 | 29.3 | 9.4 | 21.8 | 89.8 | 45.5 |
| ST9025*LR28 | 3.078 | 29.3 | 9.3 | 27.9 | 95.7 | -- |
| ST9025*LR28 | 3.002 | 30.1 | 8.2 | 15.1 | 83.4 | 45.5 |
| ST9025*LR28 | 3.139 | 30.3 | 9.7 | 28.4 | 98.7 | 46.6 |
| ST9025*LR28 | 3.075 | 30.9 | 10.0 | 13.5 | 85.2 | 44.5 |
| ST9025*LR28 | 3.180 | 31.1 | 10.4 | 29.2 | 101.8 | -- |
| ST9025*LR28 | 3.175 | 31.4 | 10.2 | 35.4 | 108.5 | 43.3 |
| ST9025*LR28 | 3.081 | 32.5 | 9.1 | 25.9 | 100.1 | 43.5 |
| ST9025*LR28 | 3.258 | 32.6 | 9.1 | 28.0 | 102.2 | 43.3 |
| ST9025*LR28 | 3.110 | 32.8 | 10.2 | 21.6 | 97.3 | -- |
| ST9025*LR28 | 3.093 | 33.2 | 11.1 | 19.5 | 97.1 | 43.4 |
| ST9025*LR28 | 3.177 | 33.5 | 11.6 | 27.6 | 106.1 | 46.2 |
| ST9025*LR28 | 3.026 | 33.8 | 12.1 | 21.3 | 101.0 | 44.1 |
| ST9025*LR28 | 3.133 | 34.2 | 9.8 | 21.0 | 99.2 | 44.1 |
| ST9025*LR28 | 3.159 | 36.2 | 11.3 | 23.0 | 106.7 | 43.0 |
| ST9025*LR28 | 3.006 | 36.4 | 11.6 | 24.3 | 108.7 | 43.7 |
| ST9025*LR28 | 3.012 | 37.5 | 10.5 | 18.4 | 103.8 | 46.0 |
| ST9026*LR28 | 4.137 | 16.1 | 6.0 | 16.4 | 54.7 | 44.9 |
| ST9026*LR28 | 4.094 | 21.5 | 8.2 | 31.9 | 83.1 | 46.4 |
| ST9026*LR28 | 4.064 | 22.5 | 8.1 | 22.3 | 75.3 | 44.8 |
| ST9026*LR28 | 4.225 | 22.5 | 8.5 | 29.4 | 82.8 | 45.5 |
| ST9026*LR28 | 4.149 | 24.1 | 8.1 | 31.8 | 88.1 | -- |
| ST9026*LR28 | 4.065 | 24.3 | 9.3 | 17.3 | 75.2 | 44.8 |
| ST9026*LR28 | 4.044 | 24.8 | 8.3 | 29.4 | 87.4 | 46.0 |
| ST9026*LR28 | 4.012 | 25.0 | 8.2 | 19.8 | 78.0 | 45.9 |
| ST9026*LR28 | 4.099 | 25.0 | 8.7 | 35.2 | 94.0 | 45.7 |
| ST9026*LR28 | 4.150 | 25.4 | 8.9 | 26.7 | 86.4 | 43.4 |
| ST9026*LR28 | 4.119 | 26.0 | 8.3 | 24.9 | 85.2 | 46.7 |
| ST9026*LR28 | 4.212 | 26.1 | 8.2 | 27.6 | 87.9 | -- |
| ST9026*LR28 | 4.266 | 26.1 | 9.3 | 27.7 | 89.3 | 44.5 |
| ST9026*LR28 | 4.098 | 26.4 | 8.6 | 33.1 | 94.3 | 47.7 |
| ST9026*LR28 | 4.026 | 26.5 | 9.6 | 24.7 | 87.3 | 45.1 |
| ST9026*LR28 | 4.210 | 26.8 | 8.4 | 32.5 | 94.5 | 45.1 |
| ST9026*LR28 | 4.170 | 26.9 | 8.6 | 26.8 | 89.2 | 43.1 |
| ST9026*LR28 | 4.031 | 26.9 | 8.8 | 28.6 | 91.2 | 44.8 |
| ST9026*LR28 | 4.046 | 27.2 | 8.3 | 26.0 | 88.6 | 42.7 |
| ST9026*LR28 | 4.055 | 27.3 | 8.9 | 30.0 | 93.5 | 45.7 |
| ST9026*LR28 | 4.224 | 27.6 | 9.3 | 28.1 | 92.6 | 44.8 |
| ST9026*LR28 | 4.274 | 28.0 | 8.7 | 22.1 | 86.8 | 45.3 |
| ST9026*LR28 | 4.096 | 28.0 | 9.5 | 24.7 | 90.2 | 45.9 |
| ST9026*LR28 | 4.118 | 28.7 | 9.3 | 17.7 | 84.4 | 47.0 |
| ST9026*LR28 | 4.063 | 28.7 | 10.1 | 17.8 | 85.3 | 45.6 |
| ST9026*LR28 | 4.184 | 29.1 | 9.1 | 34.0 | 101.3 | 45.7 |
| ST9026*LR28 | 4.223 | 29.3 | 9.9 | 31.6 | 100.0 | 45.2 |
| ST9026*LR28 | 4.196 | 29.5 | 8.9 | 21.3 | 89.3 | 46.8 |
| ST9026*LR28 | 4.180 | 29.5 | 9.5 | 29.7 | 98.1 | 44.4 |
| ST9026*LR28 | 4.214 | 30.3 | 8.6 | 31.0 | 100.2 | 43.1 |
| ST9026*LR28 | 4.093 | 30.6 | 9.7 | 26.9 | 97.9 | 43.1 |
| ST9026*LR28 | 4.190 | 30.7 | 9.3 | 30.2 | 101.0 | 45.3 |
| ST9026*LR28 | 9.217 | 31.0 | 9.6 | 30.1 | 101.7 | 46.0 |
| ST9026*LR28 | 4.104 | 32.0 | 10.3 | 25.7 | 100.0 | 44.8 |
| ST9026*LR28 | 4.290 | 32.6 | 10.1 | 30.7 | 106.1 | 46.8 |
| ST9026*LR28 | 4.148 | 32.8 | 9.7 | 27.9 | 103.2 | 45.3 |
| ST9026*LR28 | 4.220 | 33.0 | 10.8 | 31.1 | 107.9 | 44.2 |
| ST9026*LR28 | 4.209 | 33.2 | 10.8 | 19.8 | 96.9 | 47.9 |
| ST9026*LR28 | 4.256 | 33.3 | 10.2 | 31.7 | 108.4 | 46.7 |
| ST9026*LR28 | 4.112 | 33.6 | 10.2 | 18.6 | 96.0 | 45.7 |
| ST9026*LR28 | 4.262 | 34.0 | 10.8 | 21.0 | 99.8 | 45.7 |
| ST9026*LR28 | 4.079 | 34.2 | 10.3 | 25.9 | 104.6 | 45.5 |
| ST9026*LR28 | 4.126 | 34.3 | 10.5 | 23.3 | 102.5 | 48.1 |
| ST9026*LR28 | 4.294 | 34.4 | 10.6 | 24.1 | 103.4 | 43.0 |
| ST9026*LR28 | 4.087 | 35.0 | 9.9 | 23.1 | 103.1 | 44.2 |
| ST9026*LR28 | 4.288 | 35.1 | 11.3 | 16.6 | 98.1 | 44.9 |
| ST9026*LR28 | 4.075 | 35.3 | 9.9 | 21.9 | 102.3 | 46.0 |
| ST9026*LR28 | 4.142 | 35.9 | 11.9 | 18.7 | 102.4 | 46.2 |
| ST9026*LR28 | 4.238 | 36.4 | 12.2 | 16.6 | 101.6 | 45.7 |
| ST9026*LR28 | 4.277 | 36.9 | 11.6 | 19.4 | 104.7 | 46.0 |
| ST9026*LR28 | 4.066 | 41.1 | 12.4 | 15.3 | 109.9 | 45.7 |

| | | | | | | |
|---|---|---|---|---|---|---|
| STC = Stachyose content GAL = Galactinol content PRO = Protein content RAF = Raffinose content RSAC = Total Raffinose Saccharide Content | | | | | | |

Upon sorting the F2 lines from four different elite*LR28 crosses by stachyose Content (Table 5), it became apparent that certain crosses produced segregants with lower stachyose and lower total raffinose saccharide content than did other crosses. By calculating the average effect of an elite parent upon segregants that were homozgous for stc1a, it was observed that the average stachyose content of stc1a/stc1a segregants from the crosses A4715*LR28 or X3337*LR28 was significantly lower than the average stachyose content of stc1a/stc1a segregants from the crosses ST9025*LR28 or ST9026*LR28 (Table 6). Means were considered statistically significant if the ranges defined by their standard errors (SEM's) did not overlap. F2 lines that were very low in stachyose content (i.e., less than 19 µmol/g) were generally lines that were also lowest in total raffinose saccharide content (i.e., less than 80 µmol/g - see Table 5).

**TABLE 6**

| Seed Component Means for F2 Lines Homozygous for Allele stc1a and Grouped by Pedigree | | | | | | | | | |
|---|---|---|---|---|---|---|---|---|---|
| Pedigree | Number of F2 Lines | Stachyose | | Raffinose | | Galactinol | | Total Raffinose Saccharides | |
| | | Mean | SEM | Mean | SEM | Mean | SEM | Mean | SEM |
| | | µmol/g, as | | | | | | | |
| A4715*LR28 | 45 | 22.5 | 0.9 | 8.1 | 0.2 | 33.3 | 1.1 | 86.5 | 1.7 |
| X3337*LR28 | 54 | 22.9 | 0.7 | 8.6 | 0.2 | 28.6 | 0.9 | 82.9 | 1.2 |
| ST9025*LP28 | 42 | 27.4 | 0.8 | 8.9 | 0.2 | 23.1 | 0.8 | 86.7 | 1.9 |
| ST9026*LR28 | 51 | 29.4 | 0.7 | 9.5 | 0.2 | 25.5 | 0.8 | 93.8 | 1.4 |

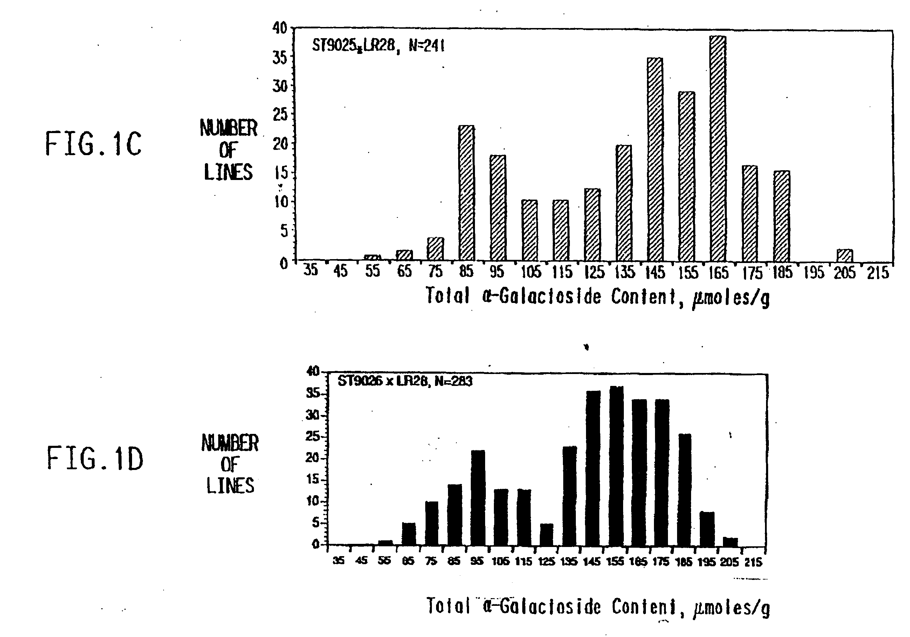

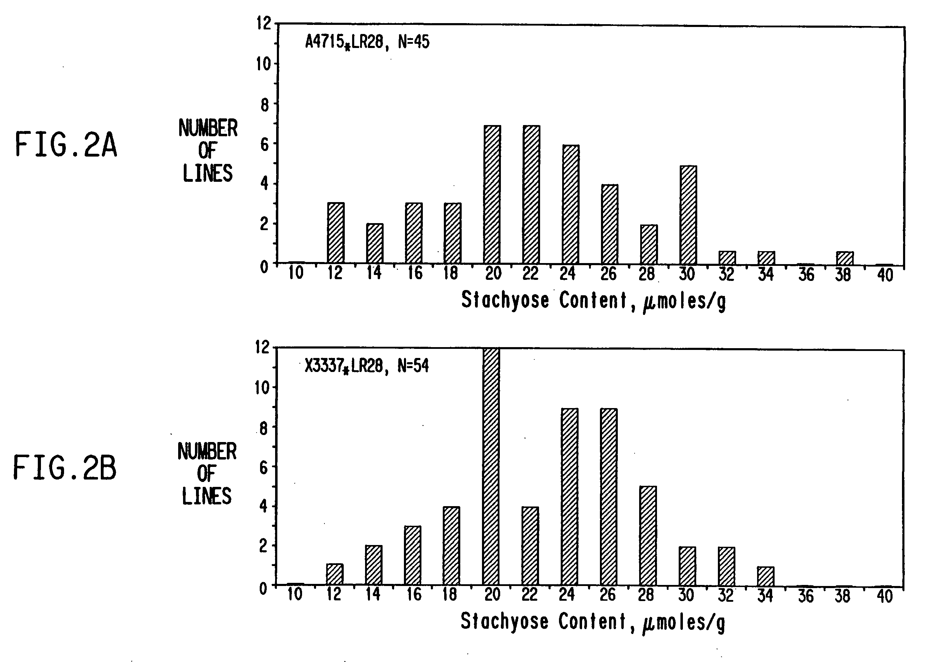

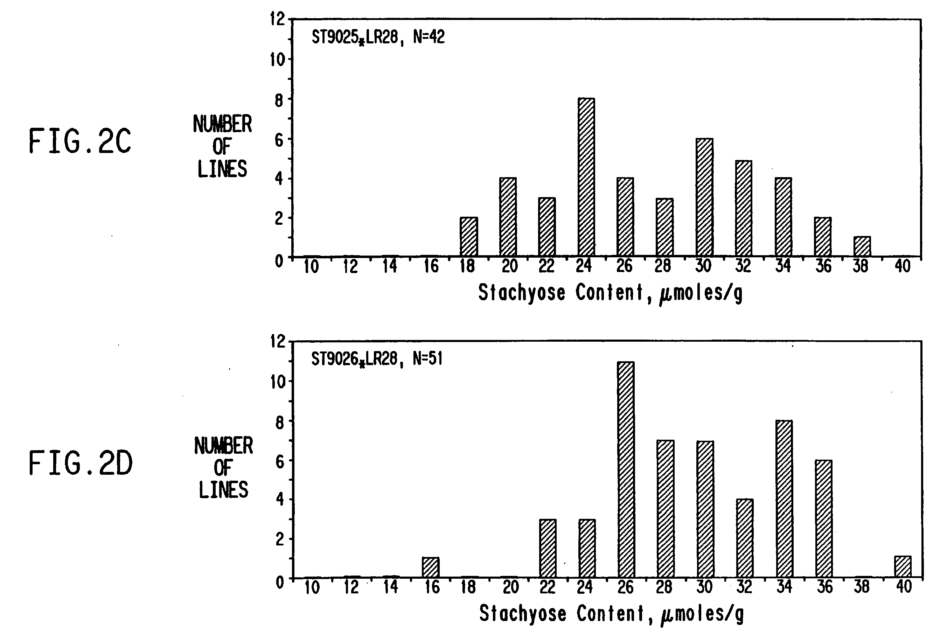

This effect of the elite parent on the expression of stc1a is best explained by other genes that modify the expression of stc1a. The segregation of modifiers of stc1a can be visualized in Figures 2 (A-D) which plots the frequency distribution of stachyose content among the "low" segregants from Figures 1(A-D) (i.e. among stc1a homozygotes). Although all segregants have the low stachyose phenotype characteristic of stc1a homozygotes, it is apparent that a class of "ultra-low" segregants (less than 19 µmol/g) is more prevalent among segregants from the A4715*LR28 and X3337*LR28 crosses than among segregants from the ST9025*LR28 and ST9026*LR28 crosses. This is most readily explained by presence of independent genetic modifier(s) present in A4715 and X3337 that modify the expression of stc1a.

According to Figures 2 (A-D), Applicants hypothesize that segregants from A4715*LR28 and X3337*LR28 containing less than 19 µmol/g stachyose are homozygous for both stc1a and a modifier of stc1a. Assuming independent assortment of stc1a and the modifier, one would expect 1/4 of the preselected stc1a homozygotes to also be homozygous for the modifier gene. Segregation among stc1a homozygotes for the "ultra-low" to "low" stachyose phenotype in these two crosses fit a 1 to 3 segregation ratio that would be expected for the segregation of single gene modifier that is recessive to codominant in gene action.

**TABLE 7**

| Segregation for Stachyose Content Among sct1a Homozygotes - Evidence of Genetic Modifiers of stc1a | | | | |
|---|---|---|---|---|
| Cross | Segregation Class | | χ² For 1:3 Ratio | Statistical Significance |
| | "ultra-low" | | "low" | |
| A4715*LR28 | 11 | 34 | 0.01 | ns |
| X3337*LR28 | 10 | 44 | 1.21 | ns |

These data provide evidence that elite lines can possess genes that modify the expression of stc1a. Since two out of four elite lines tested contained such modifiers, it is expected that other modifiers of stc1a will be common and will be discovered as the stc1a allele is recombined into a variety of other genetic backgrounds. Since researchers (including Applicants) have not found significant genetic variation among elite lines for raffinose saccharide content, such modifiers may have little or no effect in the absence of stc1a. The discovery of stc1a by Applicants will therefore permit the discovery and application of otherwise quiescent genes.

### Development of Lines With Low Raffinose Saccharide Content in Combination With Other Valuable Seed and Agronomic Traits

Protein content among the individual F2 lines with a total raffinose saccharide content less than 110 µmoles/g (as is) ranged from about 41 to greater than 48% (DB) (Table 5). This exceeds the range of protein content observed among plants within the elite lines A4715 (42.7% to 44.7% range) and X3337 (41.1% to 42.6% range) that were grown in the same environment as the F2 lines. LR28 contributed genes for high protein in addition to stc1 a to the segregating population. The correlation between total α-galactoside content and protein content within each of the four segregating F2 populations were as follows: R² = 0.029, 0.000, 0.014, and 0.004 for the crosses A4715*LR28, X3337*LR28, ST9025*LR28, and ST9026*LR28 respectively. None of these correlations were statistically significant. This is good evidence that there is no association between protein content and the stc1a allele. Although the high seed protein content of LR28 segregated independently of the stc1 a gene, many F2 lines with both low raffinose saccharide content and high protein content were observed in the elite*LR28 F2 populations (Table 5).

Therefore, it is possible to develop lines with both low raffinose saccharide content and high protein content.

### EXAMPLE 2

### CREATION OF stc1b, A SOYBEAN GENE CONFERRING IMPROVED CARBOHYDRATE COMPOSITION

### Mutagenesis and Selection of Mutants

A number of soybean lines were treated with a chemical mutagen, NMU (N-nitroso-N-methylurea), in an attempt to induce mutations that lower the raffinose saccharide content of soybean seeds. Lines treated included the elite lines Williams 82 and A2543, USDA germplasm lines A5 and N85-2176, and LR13. LR13 was originally a putative mutant of Williams 82 but did not confirm as being significantly lower in raffinose saccharide content than William 82 in subsequent tests. The following protocol for the mutagenesis of LR13 is representative of the method by which the above lines were treated with NMU and advanced through subsequent generations to obtain populations that could be screened for low raffinose saccharide mutations.

Approximately 130,000 seeds (22.7 kg) of LR13 (a line essentially identical to Williams 82) were soaked in 150 L of tap water under continuous aeration for 8 hours. Aeration was accomplished by pumping air through standard aquarium "airstones" placed in the bottom of the soaking vessel. Imbibed seeds were drained and transferred to 98 L of a 2.5 mM N-nitroso-N-methylurea (NMU) solution buffered at pH 5.5 with 0.1 M phosphate buffer under continuous aeration. Seeds remained in the NMU solution for 3 h and were then put through a series of rinses to leach out the remaining NMU. For the first rinse, treated seeds were transferred to 45 L of tap water for 1 min. For the second rinse, seeds were transferred to 45 L of fresh tap water under continuous aeration for 1 h. For the third rinse, seeds were transferred to 45 L of fresh tap water under continuous aeration for 2 h. For the fourth rinse, seeds were transferred to 45 L of fresh tap water under continuous aeration. One half of the seeds were removed from the fourth rinse after 2 h (sub-population 1) while the other half of the seeds were removed from the fourth rinse after 5 h (sub-population 2). After removal from the fourth rinse, seeds were drained of exogenous water and spread out on cardboard sheets to dry off in the sun for 1 h. The imbibed M1 seeds were then field planted (Isabela, Puerto Rico, USA) in rows spaced 46 cm apart at a density of approximately 14 seeds per foot within the rows and a depth of 2.5 cm.

Two pools of M2 seeds (from sub-populations 1 and 2) were harvested in bulk from the M1 Plants. Approximately 40,000 M2 seeds from sub-population 1 and 52,000 M2 seeds from sub-population 2 were planted at Isabela, Puerto Rico, USA. Within each sub-population, 5 pods from each of 3,000 M2 plants were harvested and bulked to obtain a bulk M3 seed population. M3 bulks were planted at Isabela, Puerto Rico. At maturity, seed from 5000 M3 plants were harvested individually to obtain 5000 M3:4 lines from each sub-population.

A total of at least 8,000 M3:4 lines were screened to measure the content of raffinose saccharides using the enzymatic method described in Example 1. One M3:4 line, LR484 (derived from the LR13 mutagenesis), was selected as having a lower raffinose saccharide content than elite soybean lines.

During early June of 1991, M3:4 seeds of LR484 were planted in Newark, Delaware, USA. In the fall of 1991, the M4:5 seeds harvested from 9 individual plants of LR484 were assayed by the HPAEC/PAD method (Example 1) for carbohydrate content. Likewise, during the winter of 1991/1992, M4:5 seeds of LR484 were grown in Puerto Rico and M5:6 seeds were harvested. M5:6 seeds from 5 individual plants of LR484 were assayed by the HPAEC/PAD method for carbohydrate content. The 1991 Newark, Delaware environment and 1992 Isabela, Puerto Rico environment were referred to as the "ST91" and "IP92" environments, respectively. In addition to LR484, LR28 and elite lines were grown in both the ST91 and IP92 environments as a basis for comparison. Stachyose, raffinose, galactinol, and total raffinose saccharide content of these lines in two diverse environments was used to confirm the phenotype of LR484. Results (Table 9) indicate that LR484 Contains dramatically less stachyose, raffinose, and total raffinose saccharide content than elite lines. These results also indicate that LR484 contains heritable genetic variation for raffinose saccharide content. The differences in carbohydrate profile between LR484 and LR28 were relatively minor compared to the differences between LR484 and the elite lines. These phenotypic data alone indicated that LR484 was a potentially valuable germplasm source.

A deposit of soybean seed designated LR484, was deposited on 15 October 1992 with the American Type Culture Collection, 12301 Parklawn Drive, Rockville, Maryland, USA, 20851, and assigned the accession number ATCC 75325. The deposit was made under terms of the Budapest Treaty.

**TABLE 9**

| Confirmation of Low Raffinose Saccharide Phenotype of LR484 | | | | | | | | | | |
|---|---|---|---|---|---|---|---|---|---|---|
| Environment | Line | N | Stachyose | | Raffinose | | Galactinol Total Raffinose Saccharide | | | |
| | | | Mean | SEM | Mean | SEM | Mean | SEM | Mean | SEM |
| | | | µmol/g, as is | | | | | | | |
| ST91 | A3322 | 8 | 94.0 | 3.5 | 19.1 | 1.0 | 0.0 | 0.0 | 207.1 | 7.3 |
| ST91 | A4715 | 9 | 98.7 | 2.8 | 17.1 | 0.8 | 4.8 | 2.2 | 217.1 | 6.4 |
| ST91 | WM82 | 7 | 60.3 | 1.3 | 12.4 | 0.4 | 2.5 | 0.2 | 135.4 | 2.8 |
| ST91 | LR28 | 18 | 20.1 | 0.6 | 4.3 | 0.6 | 46.6 | 2.4 | 88.8 | 3.1 |
| ST91 | LR484 | 9 | 24.4 | 1.1 | 2.4 | 0.2 | 11.9 | 0.4 | 63.2 | 2.1 |
| IP92 | A3322 | 10 | 77.3 | 0.8 | 16.0 | 0.8 | 0.0 | 0.0 | 170.7 | 2.1 |
| IP92 | LR28 | 5 | 14.3 | 0.5 | 1.4 | 0.4 | 24.7 | 0.7 | 54.6 | 1.4 |
| IP92 | LR484 | 5 | 20.4 | 1.6 | 3.2 | 0.3 | 11.7 | 0.5 | 55.7 | 3.6 |

| | | | | | | | | | | |
|---|---|---|---|---|---|---|---|---|---|---|
| N = number of plants assayed SEM = standard error or the mean ST91 = Summer 1991 at Stine Farm, Newark, DE, USA IP92 = Winter 1992 at Isabela, PR, USA | | | | | | | | | | |

### Allelism Test

During the summer of 1991 in Newark Delaware, LR484 was crossed with LR28 to determine if the mutation conferring low raffinose saccharide content in LR484 was allelic to stc1a. F1 seeds of the cross LR28*LR484 were harvested in early September of 1991 and planted in the greenhouse to produce F2 seeds that were harvested in December of 1991. After harvest, F2 seeds were promptly shipped to and planted in Isabela, Puerto Rico along with parental check seeds of LR28, LR484, and a conventional line A3322, used as an experimental control. F2-derived F3 seeds (F2:3 seeds) from each F2 plant and seeds from parental check plants were harvested in early 1992 from the IP92 environment and used for the allelism study. Seeds from F2 and check plants were assayed for total α-alactoside content by the enzymatic method described in Example 1.

If the mutation in LR484 is allelic with the stc1a allele from LR28, one would expect no segregation among F2 progeny from the LR28*LR484 cross. In addition, one would expect all such F2 plants to produce seeds with low raffinose saccharide content (within the range of the parent lines). If LR484 and LR28 contained nonallelic and independently segregating genes for low raffinose saccharide content, one would expect at least some recombinant F2 plants that produce seed with normal (high) raffinose saccharide content.

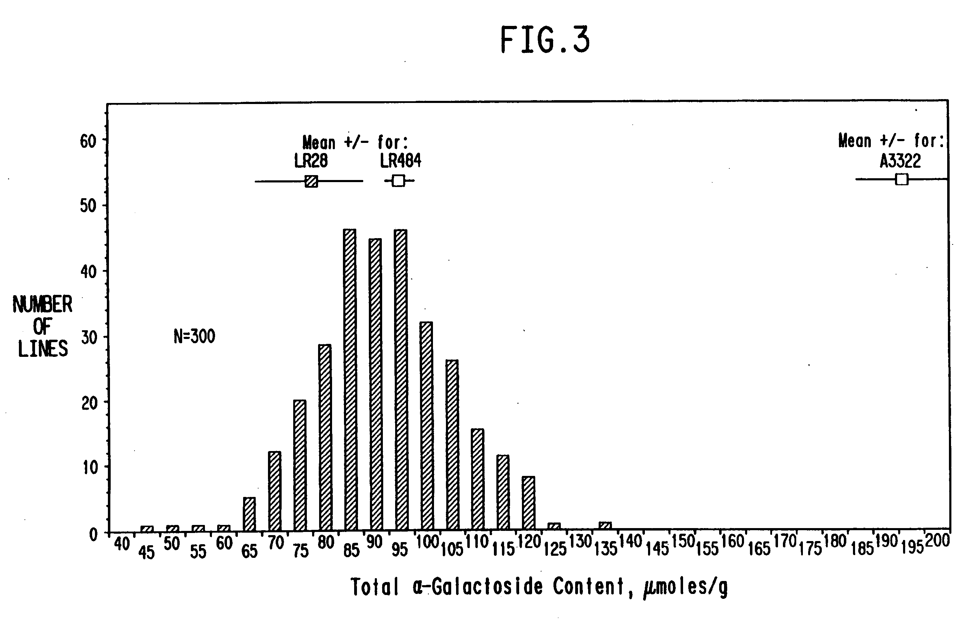

The unimodal distribution of F2 phenotypes from the LR28*LR484 cross (Figure 3) clustered around a mean total α-galactoside content of ca. 90 µmol/g and covered a range of ca. 45 µmol/g to 125 µmol/g. This range was consistent with the range displayed by stc1 a homozygotes from the elite*LR28 crosses (the "low" mode of the bimodal phenotypic distributions of Figures 1A-D). None of the F2 plants produced seed that were in the α-galactoside range of normal soybean plants (the elite line A3322 had a mean α-galactose content of 191 +/- 9 in the same IP92 environment). Results from the LR28*LR484 cross are therefore consistent with a lack of segregation at the Stc1 locus. This indicates that LR484 and LR28 contain allelic genes that both confer the low raffinose saccharide phenotype. The allele in LR484 conferring low raffinose saccharide is herein named "stc1b". Although the check plants of LR28 and LR484 were all low in α-galactoside content, LR484 plants were slightly higher in α-galactoside content than LR28 (Figure 3). When the two lines were compared using the more informative HPAEC/PAD analysis (Table 9), small relatively insignificant differences were also observed. These small differences between LR484 and LR28 may be either a consequence of genetic background differences that modify stc1x expression or a difference between the stc1a and stc1b in terms of gene action. The phenotypic range of stc1x homozygotes from a cross generally exceeds the range displayed among plants of a given stc1 x inbred line (Figure 3). This is likely due to the segregation of background genes (in crosses) that have a relatively minor affect on raffinose saccharide content. Inbred lines would be more or less homogeneous (non-segregating) for such background genes.

### EXAMPLE 3

### UTILIZATION OF APPLICANTS GENETIC VARIATION FOR THE PRODUCTION OF IMPROVED SOY PROTEIN PRODUCTS

### Preparation of Soy Protein Products: Full-Fat Flakes, Defatted White Flakes, and Desolventized, Toasted Meals

Soybean meals were prepared from 5 elite and 11 stc1x lines under laboratory or pilot plant conditions from field grown samples that ranged from Ca. 5 to 500 pounds. The processing conditions employed were designed to mimic closely those used by commercial manufacturers of defatted flakes and desolventized, toasted soybean meals [see JAOCS (1981) Vol 58, Number 3]. Different processing equipment was used, depending on the quantities of seed available. Commercial conditions can not be mimicked exactly due to inherent differences in the equipment used in a laboratory setting compared to those found in a commercial facility. Nevertheless, the conditions employed, if not the exact equipment, approximate those used commercially.

For small batches of grain (less than 10 pounds) soybeans were tempered to between 8 and 10% moisture at 54°C and then cracked in a 10" x 12" cracking roller mill (Ferrell-Ross, Oklahoma City, OK, USA). Rolls were 10" in diameter and 12" wide and had 8 and 10 grooves/inch (sawtooth), turning at about 700 and 1100 rpm (revolutions per minute), respectively. The gap between the rolls was set to be about 0.05". Hulls were removed by air aspiration using a 'Carter Dockage Tester' (Simon-Day, Winnipeg, Manitoba). Samples were passed through twice to insure hull removal.
Alternatively, hulls were removed with a Kice multi-pass aspirator (Kice Industries, Wichita, KS, USA). The dehulled meats were flaked using 18" x 18" flaking rolls (E. R. and F. Turner Ltd.), turning at 290 and 285 rpm respectively, and set with a gap of 0.003" (minimum). Full-fat flakes were extracted for 6 h in a soxhlet extractor (12 or 20 L, depending on sample size). Following extraction, the defatted meals were air desolventized for a minimum of 48 h at room temperature in a fume hood. Following desolventization, the moisture content of the defatted meals were determined gravimetrically [AOCS Method Ba 2a-38], water was added to bring the sample to 10% moisture content, and the resultant sample was mixed in a 6 L Waring blender. Following mixing, the samples was transferred to a sealed plastic bucket and allowed to equilibrate overnight. Defatted meals were then toasted to have a KOH nitrogen solubility within the range of 75 ± 10% [Araba and Dale (1990) Poultry Science 69:76-83] using a combination of heating in a 650 watt microwave oven with a 4000v DC magnetron (2450 MHz) and a convection oven set to 115°C. The residence time in the microwave oven was typically 4.5 min and 45 min in the convection oven, but some samples were heated for longer periods of time to insure similar KOH nitrogen solubility among samples.

Alternatively, defatted flakes were prepared by passing 49°C hexane over a bed of flakes within a stainless steel vessel using a solvent:flake ratio of 6:1. Six cycles of extraction were used to reduce the oil content to less than the standard specification of 0.5%. Following extraction, the flakes were allowed to desolventize overnight at room temperature in a fume hood. Following desolventization, moisture was added at a ratio of 160 mL/kg flakes. The flakes were mixed during the addition of water to insure uniform hydration and were then tempered for 5 min at 27°C. The tempered flakes were toasted at 149°C for 45 min in a heated jacket nut roaster. Following the roasting period, the vessel was opened for 5 min to allow any excess moisture that may have accumulated during the toasting to escape. At this stage the desolventized, toasted meals were ready for use in animal feeding studies.

### Nitrogen-Corrected, True Metabolizable Energy Assays

Depending on the particle size distribution toasted meals were fed directly or as 1:1 mixtures with ground corn in order to facilitate administration of the materials to the test birds. The mixtures were assayed for Nitrogen-orrected, True Metabolizable Energy content (TME_{N}) using a modification of the protocol described previously [Dale and Fuller (1987) Special Report No. 319, University of Georgia College of Agriculture, Cooperative Extension Service). In the case of samples fed as 1:1 mixtures with ground corn, the ground corn alone was also precision fed in order to correct for the metabolizable energy of this portion of the mixture. Single comb, white leghorn cockerels, placed in individual cages with raised wire floors were fasted for 24 h prior to the initiation of the study. Test material was administered directly into the crop and excreta were quantitatively collected for 48 h into plastic trays placed under each cage. Depending on the amount of material available for testing, nine or ten birds were used for each test material and a separate group were fasted throughout the study to correct for endogenous losses. Water was provided ad libitum throughout the study. Following the collection period, the excreta were dried in a forced air oven at 65°C and and the energy content of the excreta, as well as the defatted meals was determined by bomb calorimetry. Moisture content of the excreta and the defatted meals was determined using AOCS Method Ba 2a-38. Gross energy content of the excreta and defatted meals were determined using a Parr bomb calorimeter. Gross energy content and TME_{N} of the defatted meals were expressed on a dry matter basis to normalize small differences in moisture seen among samples. The efficiency of utilization of gross energy content was determined from the quotient of TME_{N} and gross energy. The results obtained from the animal feeding studies were subjected to analysis of variance.

Defatted meals from stc1x lines were found to have significantly higher (ca 12%) TME_{N} and Gross Energy Utilization than meals produced from conventional cultivars (Table 10). These results clearly indicate the utility of lines Containing sct1 compared to soybeans that are currently being used in commerce.

**TABLE 10**

| Raffinose Saccharide Content, TME_{N}, Gross Energy, and Gross Energy Utilization Efficiency of Defatted Soybean Meals Prepared from Conventional and stc1x Lines | | | | | | |
|---|---|---|---|---|---|---|
| Genotype | Number of Lines | Stachyose | Total Raffinose Saccharides | TME_{N} | Gross Energy | TME_{N} Gross Energy |
| | | µmoles/g, as is | | | | |
| Conventional | 5 | 115.5 | 252.0 | 2688 | 4813 | 0.56 |
| stc1x | 11 | 18.8** | 70.2** | 3007** | 4813 | 0.63** |

| | | | | | | |
|---|---|---|---|---|---|---|
| **Indicates that stc1x mean is significantly higher than normal mean at p<0.01 | | | | | | |

### PREPARATION OF A PET FOOD WITH IMPROVED CARBOHYDRATE COMPOSITION

### Preparation of Desolventized, Toasted Soybean Meals

For preparation of larger quantities of material, about 450 to 500 pounds each of two samples (one a conventional variety and the second a blend of stc1 a lines) were tempered to between 8 and 10% moisture at 85°C in a Model 103 Belt-O-Matic Drier (BNW Industries, Mentone, IN, USA) and then cracked in a 10" x 12" cracking roller mill (Ferrell-Ross, Oklahoma City, OK, USA). Rolls were 10" in diameter and 12" wide and had 10 and 6 grooves/inch, turning at 587 and 417 rpm, respectively. The gap between the rolls was set to 0.022". Hulls were removed by air aspiration using a Kice multi-pass aspirator (Kice Industries, Wichita, KS, USA). The dehulled meats were heated in a 22" diameter x 16" deep, bottom agitated French Cooker (French Oil Mill Machinery, Piqua, OH, USA) for about 35 min using a steam jacket. The meats were agitated by a stirring arm during this step in the process. The final temperature of the meats was about 54°C. Following heating, the meats were flaked using 12" x 12" flaking rolls (Ferrell-Ross, Oklahoma City, OK, USA), turning at 300 and 450 rpm respectively, and set with a gap of 0.012". Full-fat flakes were extracted in a Crown Model 4 Extractor (Crown Iron Works, Minneapolis, MN, USA) using a solvent ratio of about 1.5 to 1 and a temperature of about 52°C. Following extraction, the defatted meals were desolventized in a Crown Desolventizer Toaster between 210° and 220°F to obtain optimum toasting of the desolventized meal.

Following preparation of the desolventized, toasted meals, samples of the meals were analyzed for raffinose saccharide content suing the HPAEC/PAD method described in Example 1. The results of this analysis are presented in Table 11.

**TABLE 11**

| Raffinose Saccharide Content of Unprocessed Sobyeans and Desolventized, Toasted Meals Processed from a Conventional Soybean Variety and stc1a Lines | | | |
|---|---|---|---|
| Sample | Raffinose | Stachyose | Total Raffinose Saccharide |
| | µmoles/g, as is | | |
| Whole Soybean: | | | |
| Conventional | 20.6 | 115.8 | 252.3 |
| stc1a | 3.0 | 17.1 | 74.0 |

| Soybean Meal: | | | |
|---|---|---|---|
| Conventional | 32.9 | 183.3 | 399.7 |
| stc1a | 4.8 | 23.6 | 98.4 |

### Preparation of Pet Foods With Improved Stachyose Content

Following preparation of the desolventized, toasted soybean meals, rations were formulated to prepare extruded dry, expanded foods under conditions similar to those commonly used in commercial operations. Soybean meal is a common ingredient used by many dry, expanded food manufacturers and the inclusion rate of soybean meal can vary depending on the desires of the manufacturer. As a result, extruded dry, expanded foods were prepared with two levels of inclusion of soybean meal in order to produce rations which cover the range of soybean meal inclusion percentage that is commonly observed in commercial products. The product formulas used for preparation of the pet foods are shown in Table 12.

**TABLE 12**

| Ingredient Formulas Used in the Preparation of Pet Foods with Decreased Stachyose Content | | | | |
|---|---|---|---|---|
| | Diet | | | |
| Ingredient | 1 | 2 | 3 | 4 |
| | % Inclusion | | | |
| Corn | 63.53 | 63.53 | 48.34 | 48.34 |
| Conventional Soybean Meal | 0.00 | 25.97 | 0.00 | 40.96 |
| stc1a Soybean Meal | 25.97 | 0.00 | 40.96 | 0.00 |
| Meat Meal | 6.59 | 6.59 | 6.59 | 6.59 |
| DiCal (38% Ca) | 1.76 | 1.76 | 1.50 | 1.50 |
| Calcium Carbonate | 0.36 | 0.36 | 0.36 | 0.36 |
| Salt | 0.55 | 0.55 | 0.55 | 0.55 |
| Animal Fat | 0.80 | 0.80 | 1.30 | 1.30 |
| Vitamin Premix | 0.22 | 0.22 | 0.22 | 0.22 |
| Mineral Premix | 0.11 | 0.11 | 0.11 | 0.11 |
| Choline-Cl, 60% | 0.11 | 0.11 | 0.07 | 0.07 |
| Total | 100.00 | 100.00 | 100.00 | 100.00 |

The pet foods were prepared using conditions similar to those employed in commercial operations [Rokey (1983) Feed Manufacturing Technology III, 222-237; McCulloch, (1984) US Patent 4,454,804] using a Wenger Model TX-52 single-screw extruder using a die with a 5/32" diameter orifice (Wenger Manufacturing, INC, Sabetha, KS, USA). In order to establish optimum conditions during the extrusion process, an initial run was performed using the formula employed in Diet 1 above, with the exception that a conventional soyflour was used in place of the desolventized, toasted soybean meal. Corn was ground using a Fitzmill Model D (Fitzpatrick CO, Cincinnati, OH, USA) at 4536 rpm with a screen (3.2 mm openings) and then blended with the remaining ingredients for 5 min. The final mix was then ground in the Fitzmill using a 20 mesh screen. The conditions used during the extrusion and drying process for the 4 diets are indicated in Table 13.

**TABLE 13**

| Conditions Used During Extrusion and Drying in the Manufacture Pet Foods from Conventional and stc1x Lines | | | | |
|---|---|---|---|---|
| | Diet | | | |
| Raw Material Information | 1 | 2 | 3 | 4 |
| Raw Material Moisture, mcwb | 9.60 | 9.66 | 7.58 | 9.42 |
| Raw Material Rate, pph | 210 | 210 | 210 | 210 |
| Feed Screw Speed, rpm | 20 | 20 | 20 | 20 |

| Preconditioning Information | | | | |
|---|---|---|---|---|
| Mixing Cylinder Speed, rpm | 165 | 165 | 165 | 165 |
| Steam to Mixing Cylinder, ppm | 0.16 | 0.16 | 0.16 | 0.16 |
| Water to Mixing Cylinder, ppm | 0.27 | 0.27 | 0.27 | 0.27 |
| Mixing Cylinder Temp. °F | 170 | 170 | 170 | 170 |
| Moisture Entering Extruder, mcwb | 18.47 | 19.87 | 19.32 | 19.26 |

| Extrusion Information | | | | |
|---|---|---|---|---|
| Shaft Speed, rpm | 385 | 385 | 385 | 385 |
| Motor Load, % | 18 | 17 | 19 | 18 |
| Steam Flow to Extruder, ppm | 0.13 | 0.13 | 0.13 | 0.13 |
| Water Flow to Extruder, ppm | 0.20 | 0.18 | 0.18 | 0.20 |
| Control/Temp 2nd Head, °F | cw/185 | cw/148 | cw/149 | cw/152 |
| Control/Temp 3rd Head, OF | cw/202 | cw/198 | cw/197 | cw/199 |
| Control/Temp 4th Head, °F | s/205 | s/208 | s/223 | s/229 |
| Control Temp 5th Head, °F | cw/197 | cw/188 | cw/175 | cw/194 |
| Pressure 5th Head, psi | 400 | 400 | 400 | 400 |

| Final Product Information | | | | |
|---|---|---|---|---|
| Extrudate Moisture, mcwb | 20.44 | 22.42 | 18.18 | 20.72 |
| Extrudate Rate (wet), pph | 252 | 260 | 260 | 260 |
| Extrudate Density (dry), 1bs/ft³ | 24.5 | 26.0 | 23.5 | 24.5 |
| Extrudate Density (wet), 1bs/ft³ | 24.0 | 24.0 | 23.5 | 24.5 |

| Dryer condition; | | | | |
|---|---|---|---|---|
| Dryer Model | 4800 | 4800 | 4800 | 4800 |
| Number of Sections | 3 | 3 | 3 | 3 |
| Temperature, Zone 1, °F | 230 | 230 | 230 | 230 |
| Temperature, Zone 2, °F | 230 | 230 | 230 | 230 |
| Temperature, Zone 3, °F | 230 | 230 | 230 | 230 |
| Retention Time, 1st Pass, min | 8 | 8 | 8 | 8 |
| Retention Time, 2nd Pass, min | 8 | 8 | 8 | 8 |
| Fan #1 Speed, rpm | 1200 | 1200 | 1200 | 1200 |
| Fan #2 Speed, rpm | 1200 | 1200 | 1200 | 1200 |
| Fan #3 Speed, rpm | 1200 | 1200 | 1200 | 1200 |

| | | | | |
|---|---|---|---|---|
| cw = cooling water s = steam mcwb = moisture content wet basis pph = pounds per hour ppm = pounds per minute rpm = revolutions per minute psi = pounds per in² rpm = revolutions per minute | | | | |

Using the formulas and processing conditions described above, the four pet foods were prepared.

Meals prepared from the stc1a line and the conventional variety were observed to be completely compatible with the equipment used in the preparation of the dry, expanded food. Following preparation of the dry, expanded foods, samples were analyzed for their raffinose saccharide content using the HPAEC/PAD method described in Example 1. In addition, several commercially available dry, expanded dog foods were analyzed for their raffinose saccharide content for use in comparison to the pet foods prepared in the instant invention. The composition of the pet foods are shown in Table 14. The results indicated that even when used at soybean meal inclusion rates of Ca. 41%, pet foods prepared from soybean meal from stc1a lines contained substantially less raffinose, stachyose and total raffinose saccharides than those from the conventional variety and the commercial pet foods. Meals, flours and grits prepared from soybean have application in a broad number of pet food products including, but not limited to dry, semi-moist and canned foods. For example, these soy protein products are also used in the manufacture of snack foods for pets. The soy protein products derived from stc1 x lines described in the present invention should have broad applicability in all of the pet products that currently utilize soy protein products from conventional soybeans.

**TABLE 14**

| Raffinose Saccharide Content of Pet Foods Produced from Soybean Meal Processed from stc1 a Lines | | | |
|---|---|---|---|
| Pet Food | Raffinose | Stachyose | Total Raffinose Saccharide |
| | µmoles/g, db | | |
| Diet 1 (stc1a) | 2.6 | 5.7 | 25.2 |
| Diet 3 (stc1 a) | 3.4 | 9.6 | 38.1 |
| Diet 2 Conventional | 6.4 | 24.0 | 56.8 |
| Diet 4 (Conventional) | 9.1 | 39.1 | 88.2 |
| Purina Dog Chow | 6.2 | 15.6 | 37.5 |
| Dealer's Choice | 18.0 | 18.0 | 46.9 |
| Kibbles & Bits | 21.2 | 21.2 | 52.1 |

### PREPARATION OF EDIBLE SOY PRODUCTS WITH IMPROVED CARBOHYDRATE COMPOSITION

In order to determine the commercial utility of soy protein products produced from stc1x x lines, an assortment of commercially available soy protein products (soy flours, soy concentrates, etc.) were purchased from local retail sources, air dried (or lyophilyzed if the commercial product contained a high water content) and analyzed for raffinose saccharide content. The precise details for the manufacture of these products is confidential and therefore unknown to the Applicants. However, it is known that many of these commercial products have been processed, in part, specifically to reduce the raffinose saccharide content of the conventional soybean component used.

Defatted soy flakes were prepared from about 3 pound samples of stc1 a lines using the same processing conditions used for small batches as described above. The resultant white flakes were then analyzed for raffinose saccharide content using the HPAEC/PAD method described in Example 1. The results of are presented in Table 15.

**TABLE 15**

| Raffinose Saccharide Content of White Flakes Prepared From stc1a Lines | | | |
|---|---|---|---|
| Sample | Raffinose | Stachyose | Total Raffinose Saccharide |
| | µmoles/g, as is | | |
| Whole Soybean: | | | |
| AI9181 (stc1a) | 1.8 | 11.1 | 58.1 |
| ST9181 (stc1a) | 1.5 | 11.6 | 60.1 |
| ST911458 (stc1a) | 1.9 | 11.1 | 58.5 |
| D92-08 (conventional) | 12.6 | 65.2 | 149.4 |
| D92-10 (conventional) | 15.8 | 85.8 | 187.8 |

| White Flakes: | | | |
|---|---|---|---|
| Al9181 (stc1a) | 2.3 | 14.0 | 79.7 |
| ST9181 (stc1a) | 3.3 | 18.3 | 93.5 |
| ST911458 (stc1a) | 1.7 | 11.5 | 61.3 |
| D92-08 (Conventional) | 18.8 | 97.8 | 214.4 |
| D92-10 (Conventional) | 16.5 | 80.4 | 177.4 |
| Commercial Soy Protein Products: | | | |

| | µmoles/g, db | | |
|---|---|---|---|
| Central Soya, Ft. Wayne, IN, USA | | | |
| SoyaFluff (flour) | 33.2 | 131.7 | 296.5 |
| Centrex (textured flour) | 32.1 | 124.8 | 281.7 |
| Promocaf (concentrate) | 6.5 | 49.8 | 106.0 |
| Response (textured conc.) | 4.8 | 35.3 | 75.4 |

| Solnuts, Inc., Hudson, IA, USA | | | |
|---|---|---|---|
| Halves | 12.7 | 47.8 | 108.3 |
| Soyflour (Full-Fat) | 15.5 | 47.1 | 109.7 |
| Diced | 17.1 | 60.5 | 138.0 |

As was seen previously with preparation of desolventized, toasted meals, white flakes prepared from stc1a lines displayed a substantially improved raffinose saccharide content compared to those prepared from conventional lines. These results indicate that white flakes produced from stc1x lines should have broad commercial applicability for a wide variety of soy protein products currently produced using white flakes as a starting material. These include, but are not limited to, flours and concentrates. This is supported by the observation in the present invention that white flakes produced from stc1a lines displayed a superior raffinose saccharide content to several commercially available soyflours (both full-fat and textured) and soy protein concentrates. The white flakes from sty1a lines were even found to have a lower raffinose saccharide content than that seen in even more highly processed soy protein concentrates made from conventional soybeans.

## Claims

1. Soybeans with a genotype that confers a heritable phenotype of seed stachyose content of less than 30 µmol/g (based on undried seed), said soybeans being non-viable as a result of mechanical processing such as dehulling, cracking or grinding, wherein said soybeans are obtainable from progeny lines prepared by a method comprising:
(a) crossing a soybean plant comprising a stc1x allele, wherein said plant is of a line that has a genotype at the Stc1 locus that confers a phenotype of a seed stachyose content of less than 30µmol/g (based on undried seed), with an agronomically elite soybean parent which does not comprise said allele, to yield a F1 hybrid;
(b) selfing the F1 hybrid for at least one generation; and
(c) identifying the progeny of step (b) homozygous for the stc1x gene and capable of producing seed having a stachyose content of less than 30 µmol/g (based on undried seed).

2. Soybeans of claim 1 wherein said stachyose content of said soybeans is less than 15 µmol/g (based on undried seed).

3. Soybeans of claim 1 or 2 having a heritable phenotype of a seed total raffinose saccharide content of less than 110 µmol/g (based on undried seed).

4. Soybeans of any one of claims 1 to 3 further comprising a seed protein content of greater than 42%.

5. Method of using soybeans of any one of claims 1 to 4, the method comprising further processing said soybeans to obtain a desired soy product by means known *per se.*

6. Method of making a soy protein product comprising processing soybeans of any one of claims 1 to 4 by means known *per se.*

7. A method of making a full fat soy protein product comprising:
(a) removing the meats from the hulls of cracked soybeans as defined in any one of claims 1 to 4;
(b) flaking the meats obtained in step (a) to obtain a desired flake thickness;
(c) heat-denaturing the flakes obtained in step (b) to obtain a desired Nitrogen Solubility Index; and
(d) grinding the denatured flakes of step (c) to obtain a desired particle size.

8. A method of making a defatted soy protein product comprising:
(a) removing the meats from the hulls of cracked soybeans as defined in any one of claims 1 to,4;
(b) flaking the meats obtained in step (a) to obtain a desired flake thickness;
(c) contacting the full fat flakes obtained in step (b) with a solvent to extract oil from the flakes to a desired content level;
(d) heat-denaturing the defatted flakes obtained in step (c) to obtain a desired nitrogen solubility; and
(e) grinding the denatured, defatted flakes obtained in step (d) to obtain a desired particle size.

9. A method of making a soy protein concentrate product comprising:
(a) removing the meats from the hulls of cracked soybeans as defined in any one of-claims 1 to 4;
(b) flaking the meats obtained in step (a) to obtain a desired flake thickness;
(c) contacting the full fat flakes obtained in step (b) with a first solvent to extract oil from the flakes to a desired oil content level;
(d) contacting the defatted flakes obtained in step (c) with a second solvent to obtain a soy protein concentrate product with a protein content (6.25 x N) of not less than 65% (dry basis).

10. The method of claim 9, wherein the second solvent is an aqueous alcohol solution from 55 to 90% and wherein the soy protein concentrate product obtained in step (d) has a protein content (6.25 x N) of not less than 70% (dry basis).

11. The method of claim 9, wherein the second solvent is an acidic solution of pH 4 - pH 5.

12. A method of making an isoelectric soy protein isolates product comprising:
(a) removing the meats from the hulls of cracked soybeans as defined in any one of claims 1 to 4;
(b) flaking the meats obtained in step (a) to obtain a desired flake thickness;
(c) contacting the full fat flakes obtained in step (b) with a first solvent to extract oil from the flakes to a desired oil content level;
(d) contacting the defatted flakes obtained in step (c) with an aqueous solution of pH 8 - pH 9;
(e) separating the soluble and insoluble fractions of the product of step (d) by physical means;
(f) adjusting the pH of the soluble fraction obtained in step (e) to obtain a protein precipitate;
(g) separating the protein precipitate of step (f) from the soluble fraction by physical means to obtain a soy protein isolate;
(h) washing the product of step (g); and
(i) spray-drying the washed product of step (h) to obtain an isoelectric soy protein isolate product.

13. The method of claim 12 further comprising mixing the isoelectric soy protein isolate product obtained in step (i) with sufficient alkali to increase the solubility of the product to a desired level.

14. A soy protein product made from a soybean as defined in any one of claims 1 to 4 wherein said product is soy milk having a stachyose concent of less than 45 µmol/g (as is).

15. An undenatured, defatted soy protein product made from a soybean as defined in any one of claims 1 to 4 having a stachyose content of less than 45 µmol/g (as is).

16. A heat-processed, desolventized and toasted soy protein product made from a soybean as defined in any one of claims 1 to 4 having a stachyose content of less than 45 µmol/g (as is); and having a true metabolizable energy content of greater than 2850 Kcal/kg (dry basis).

17. A heat-processed, defatted flash-desolventized soy protein product made from a soybean as defined in any one of claims 1 to 4 having a stachyose content of less than 45 µmol/g (as is).

18. A soy protein concentrate product having a protein Content (6.5 x N) of not less than 65% (dry basis) produced by the method comprising:
(a) removing the meats from the hulls of cracked soybeans as defined in any one of claims 1 to 4;
(b) flaking the meats obtained in step (a) to obtain a desired flake thickness;
(c) contacting the full fat flakes obtained in step (b) with a first solvent to extract oil from the flakes to a desired content level;
(d) contacting the defatted flakes obtained in step (c) with a second solvent to obtain a soy protein concentrate product with a protein content (6.25 x N) of not less than 65% (dry basis).

19. A pet food product having a soybean inclusion rate of between 25 and 41% and a total stachyose content of less than 10 µmol/g (dry basis) wherein said soybean is as defined in any one of claims 1 to 4.

20. A method for producing a soybean protein product with a reduced stachyose content comprising:
(a) crossing an agronomically elite soybean line with the mutant soybean line LR28 or LR484 having a genotype that confers a heritable phenotype of seed stachyose content of less than 30 µmol/g (based on undried seed);
(b) screening the seed of progeny plants obtained from step (a) for a seed stachyose content of less than 30 µmol/g (based on undried seed); and
(c) processing the seed selected in step (b) to obtain the desired soybean protein product.

21. A method of using a soybean having a genotype at the Stc1 locus that confers a phenotype of a seed stachyose content of less than 30 µmol/g (based on undried seed) to produce progeny lines, the method comprising:
(a) crossing a soybean plant comprising a stc1x allele with an agronomically elite soybean parent which does not comprise said allele, to yield a F1 hybrid;
(b) selfing the F1 hybrid for at least one generation; and
(c) identifying the progeny of step (b) homozygous for the stc1x gene and capable of producing seed having a stachyose content of less than 30 µmol/g (based on undried seed).

22. The method of claim 21 including the steps of obtaining said soybean plant comprising a stc1x allele lower case by mutagenesis and selection.

23. The method of claim 21 wherein progeny is identified which is capable of producing seed having a stachyose content of less than 15 µmol/g (based on undried seed).

## Patentansprüche

1. Sojabohnen mit einem Genotyp, der einen erblichen Phänotyp eines Samenstachyosegehalts von weniger als 30 µmol/g (basierend auf nicht getrocknetem Samen) verleiht bzw. überträgt, wobei die Sojabohnen als ein Ergebnis von mechanischem Verarbeiten wie beispielsweise Entschälen bzw. Enthüllen, Spalten oder Mahlen nicht lebensfähig sind, wobei die Sojabohnen aus Nachkommenschaftslinien erhalten werden können, die durch ein Verfahren erzeugt werden, das umfasst:
(a) Kreuzen einer Sojabohnenpflanze, umfassend ein stc1x-Allel, wobei die Pflanze aus einer Linie stammt, die einen Genotyp am Stc1-Locus aufweist, der einen Phänotyp eines Samenstachyosegehalts von nicht weniger als 30 µmol/g (basierend auf nicht getrocktenem Samen) verleiht, mit einem agronomischen Elitesojabohnenelternteil, welches nicht das Allel umfasst, unter Erhalt eines F1 Hybrids;
(b) Selbstbefruchten des F1 Hybrids für mindestens eine Generation; und
(c) Identifizieren der Nachkommenschaft von Stufe (b), die als homozygot in Bezug auf das stc1x-Gen ist und fähig, Samen mit einem Stachyosegehalt von weniger als 30 µmol/g (basierend auf nicht getrocknetem Samen) zu bilden.

2. Sojabohnen nach Anspruch 1, wobei der Stachyosegehalt der Sojabohnen geringer als 15 µmol/g (basierend auf nicht getrocknetem Samen) ist.

3. Sojabohnen nach Anspruch 1 oder 2 mit einem erblichen Phänotyp eines Gesamtsamenraffinosesaccharidgehalts von weniger als 110 µmol/g (basierend auf nicht getrocknetem Samen).

4. Sojabohnen nach einem der Ansprüche 1 bis 3, ferner umfassend einen Samenproteingehalt von größer als 42%.

5. Verfahren zum Verwenden von Sojabohnen nach einem der Ansprüche 1 bis 4, wobei das Verfahren ferner Verarbeiten der Sojabohnen unter Erhalten eines gewünschten Sojaproduktes mittels *per* se bekannter Mittel umfasst.

6. Verfahren zum Herstellen eines Sojaproteinproduktes, umfassend verarbeiten von Sojabohnen nach einem der Ansprüche 1 bis 4 mittels per se bekannter Mittel.

7. Verfahren zum Herstellen eines vollfetten Sojaproteinproduktes, umfassend:
(a) Entfernen des Fleisches von den Schalen gespaltener Sojabohnen wie in einem der Ansprüche 1 bis 4 definiert;
(b) in Schichten zerlegen bzw. flockig machen des in Stufe (a) erhaltenen Fleisches unter Erhalten einer gewünschten Schichtdicke bzw. Flockendicke;
(c) Hitzedenaturieren der in Stufe (b) erhaltenen Schichten unter Erhalten eines gewünschten Stickstoff-Löslichkeits-Index; und
(d) Mahlen der denaturierten Schichten von Stufe (c) unter Erhalten einer gewünschten Teilchengröße.

8. Verfahren zum Herstellen eines entfetteten Sojaproteinproduktes, umfassend:
(a) Entfernen des Fleisches von den Schalen von gespaltenen Sojabohnen wie in einem der Ansprüche 1 bis 4 definiert;
(b) in Schichten zerlegen bzw. flockig machen des in Stufe (a) erhaltenen Fleisches, unter Erhalten einer gewünschten Schichtdicke bzw. Flockendicke;
(c) in Kontakt bringen der in Stufe (b) erhaltenen vollfetten Schichten mit einem Lösungsmittel unter Extrahieren von Öl aus den Schichten zu einem gewünwschten GEhaltspiegel;
(d) Hitzedenaturieren der in Stufe (c) erhaltenen entfetteten Schichten unter Erhalten einer gewünschten Stickstofflöslichkeit; und
(e) Mahlen der in Stufe (d) erhaltenen denaturierten, entfetteten Schichten unter Erhalten einer gewünschten Teilchengröße.

9. Verfahren zum Herstellen eines Sojaproteinkonzentratproduktes, umfassend:
(a) Entfernen des Fleisches von den Schalen zerspaltener Sojabohnen, wie in einem der Ansprüche 1 bis 4 definiert;
(b) in Schichten zerlegen bzw. flockig machen des in Stufe (a) erhaltenen Fleisches unter Erhalten einer gewünschten Schichtdicke bzw. Flockendicke;
(c) in Kontakt bringen der in Stufe (b) erhaltenen vollständig fetten Schichten mit einem ersten Lösungsmittel unter Extrahieren von Öl aus den Schichten zu einem gewünschten Ölgehaltspiegel;
(d) in Kontakt bringen der in Stufe (c) erhaltenen entfetteten Schichten mit einem zweiten Lösungsmittel unter Erhalten eines Sojaproteinkonzentratproduktes mit einem Proteingehalt (6,25 x N) von nicht weniger als 65% (Trockenbasis).

10. Verfahren nach Anspruch 9, wobei das zweite Lösungsmittel eine wässrige Alkohollösung von 55 bis 90% ist, und wobei das in Stufe (d) erhaltene Sojaproteinkonzentratprodukt einen Proteingehalt (6,25 x N) von nicht weniger als 70% (Trockenbasis) hat.

11. Verfahren nach Anspruch 9, wobei das zweite Lösungsmittel eine saure Lösung mit pH 4 - pH 5 ist.

12. Verfahren zum Herstellen eines isoelektrischen Sojaproteinisolatproduktes, umfassend:
(a) Entfernen des Fleisches von den Schalen von zerspaltenen Sojabohnen, wie in einem der Ansprüche 1 bis 4 definiert;
(b) in Schichten zerlegen bzw. flockig machen des in Stufe (a) erhaltenen Fleisches unter Erhalten einer gewünschten Schichtdicke bzw. Flockendicke;
(c) in Kontakt bringen der in Stufe (b) erhaltenen vollständig fetten Schichten mit einem ersten Lösungsmittel unter Extrahieren von Öl aus den Schichten zu einem gewünschten Ölgehaltspiegel;
(d) in Kontakt bringen der in Stufe (c) erhaltenen entfetteten Schichten mit einer wässrigen Lösung mit pH 8 - pH 9;
(e) Abtrennen der löslichen und unlöslichen Fraktionen des Produktes der Stufe (d) mit physikalischen bzw. physischen Mitteln;
(f) Einstellen des in Stufe (e) erhaltenen pH der löslichen Fraktion unter Erhalten eines Proteinniederschlags;
(g) Abtrennen des Proteinniederschlags der Stufe (f) aus der löslichen Fraktion mit physikalischen Mitteln unter Erhalten eines Sojaproteinisolats;
(h) Waschen des Produktes von Stufe (g); und
(i) Sprühtrocknen des gewaschenen Produktes von Stufe (h) unter Erhalten eines isoelektrischen Sojaproteinisolatprodukts.

13. Verfahren nach Anspruch 12, ferner umfassend Mischen des in Stufe (i) erhaltenen isoelektrischen Sojaproteinisolatproduktes mit ausreichend Alkali unter Erhöhen der Löslichkeit des Produktes auf einen gewünschten Spiegel bzw. Pegel.

14. Sojaproteinprodukt, hersgestellt aus einer Sojabohne wie in einem der Ansprüche 1 bis 4 definiert, wobei das Produkt Sojamilch mit einem Stachyosegehalt von weniger als 45 µmol/g (im Istzustand) ist.

15. Nicht denautriertes, entfettetes Sojeproteinprodukt, hergestellt aus einer Sojabohne wie in einem der Ansprüche 1 bis 4 definiert, mit einem Stachyosegehalt von weniger als 45 µmol/g (im Istzustand).

16. Wärmeverarbeitetes, entsolvatisiertes und geröstetes sojaproteinprodukt, hergestellt aus einer Sojabohne wie in einem der Ansprüche 1 bis 4 definiert mit einem Stachyosegehalt von weniger als 45 µmol/g (im Istzustand); und mit einem wahren metabolisierbaren Energiegehalt von größer als 2.850 kcal/kg (Trockenbasis).

17. Wärmeverarbeitetes, entfettetes, Flashentsolvatisiertes Sojaproteinprodukt, hergestellt aus einer Sojabohne, wie in einem der Ansprüche 1 bis 4 definert, mit einem Stachyosegehalt von weniger als 45 µmol/g (im Istzustand).

18. Sojaproteinkonzentratprodukt mit einem Proteingehalt (6,5 x N) von nicht weniger als 65% (Trockenbasis), hergestellt mithilfe des Verfahrens umfassend:
(a) Entfernen des Fleisches von den Schalen von gespaltenen Sojabohnen wie in einem der Ansprüche 1 bis 4 definiert;
(b) in Schichten zerlegen bzw. flockig machen des in Stufe (a) erhaltenen Fleisches unter Erhalten einer gewünschten Schichtdicke bzw. Flockendicke;
(c) in Kontakt bringen der in Stufe (b) erhaltenen vollfetten Schichten mit einem ersten Lösungsmittel unter Extrahieren von Öl aus den Schichten zu einem gewünschten Gehaltspiegel;
(d) in Kontakt bringen der in Stufe (c) erhaltenen entfetteten Schichten mit einem zweiten Lösungsmittel unter Erhalten eines Sojaproteinkonzentratprodukts mit einem Proteingehalt (6,25 x N) von nicht weniger als 65% (Trockenbasis).

19. Tiernahrungsprodukt mit einer Sojabohneneinschlussrate bzw. einem Sojabohnengehalt zwischen 25 und 41% und einem Gesamtstachyosegehalt von weniger als 10 µmol/g (Trockenbasis), wobei die Sojabohne wie in einem der Ansprüche 1 bis 4 definiert ist.

20. Verfahren zum Herstellen eines Sojabohnenproteinproduktes mit einem reduzierten Stachyosegehalt, umfassend:
(a) Kreuzen einer agronomischen Elitesojabohnenlinie mit einer Mutantensojabohnenlinie LR28 oder LR484 mit einem Genotyp, der einen erblichen Phänotyp eines Samenstachyosegehalts von weniger als 30 µmol/g (basierend auf nicht getrocknetem Samen) verleiht bzw. überträgt;
(b) Screenen des Samens der aus Stufe (a) erhaltenen Nachkommenpflanzen in Bezug auf einen Samenstachyosegehalt von weniger als 30 µmol/g (basierend auf nicht getrocknetem Samen); und
(c) Verarbeiten des in Stufe (b) ausgewählten Samens unter Erhalten des gewünschten Sojabohnenproteinprodukts.

21. Verfahren unter bzw. zum Verwenden einer Sojabohne mit einem Genotyp an dem Stc1 Locus, der einen Phänotyp mit einem Samenstachyosegehalt von weniger als 30 µmol/g (basierend auf nicht getrocknetem Samen) unter Herstellen von Nachkommenlinien verleiht, wobei das Verfahren umfasst:
(a) Kreuzen einer Sojabohnenpflanze, umfassend ein stc1x-Allel, mit einem agronomischen Elitesojabohnenelternteil, welches nicht das Allel umfasst, unter Erhalt eines F1 Hybrids;
(b) Selbstbefruchten des F1 Hybrids für mindestens eine Generation;
(c) Identifizieren der Nachkommenschaft von Stufe (b), die als homozygot in Bezug auf das stclx-Gen ist und fähig ist, Samen mit einem Stachyosegehalt von weniger als 30 µmol/g (basierend auf nicht getrocknetem Samen) herzustellen.

22. Verfahren nach Anspruch 21, einschließend die Stufen des Erhaltens der Sojabohnenpflanze, umfassend ein stc1x-Allel, durch Mutagenese und Selektion.

23. Verfahren nach Anspruch 21, wobei Nachkommenschaft identifiziert wird, welche fähig ist, Samen mit einem Stachyosegehalt von weniger als 15 µmol/g (basierend auf nicht getrocknetem Samen) zu bilden.

## Revendications

1. Graines de soja possédant un génotype qui confère un phénotype héritable présentant une teneur en stachyose dans la semence inférieure à 30 µmoles/g (sur la base de semence non séchée), lesdites graines de soja étant non viables à la suite d'une transformation mécanique telle qu'un décorticage, un concassage ou un broyage, lesdites graines de soja pouvant être obtenues à partir de lignées de descendance préparées par un procédé comportant les étapes consistant à :
(a) croiser une plante de soja comportant un allèle stclx, ladite plante étant d'une lignée qui possède un génotype au locus Stc1 qui confère un phénotype présentant une teneur en stachyose dans la semence inférieure à 30 µmoles/g (sur la base de semence non séchée), avec un soja parent élite du point de vue agronomique qui ne comporte pas ledit allèle, pour produire un hybride F1 ;
(b) auto-polliniser l'hybride F1 pendant au moins une génération ; et
(c) identifier la descendance de l'étape (b) homozygote pour le gène stc1x et capable de produire une semence présentant une teneur en stachyose inférieure à 30 µmoles/g (sur la base de semence non séchée).

2. Graines de soja selon la revendication 1, ladite teneur en stachyose desdites graines de soja étant inférieure à 15 µmoles/g (sur la base de semence non séchée).

3. Graines de soja selon la revendication 1 ou 2 possédant un phénotype héritable présentant la teneur totale en saccharide raffinose de la semence inférieure à 110 µmoles/g (sur la base de semence non séchée).

4. Graines de soja selon l'une quelconque des revendications 1 à 3, la teneur en protéines de la semence étant en outre supérieure à 42 %.

5. Procédé d'utilisation de graines de soja selon l'une quelconque dés revendications 1 à 4, le procédé comportant également la transformation desdites graines de soja pour obtenir un produit de soja désiré par des moyens connus en tant que tels.

6. Procédé de fabrication d'un produit de protéines de soja comportant la transformation de graines de soja selon l'une quelconque des revendications 1 à 4 par des moyens connus en tant que tels.

7. Procédé pour fabriquer un produit de protéines de soja non dégraissé, comportant les étapes consistant à :
(a) retirer les chairs des enveloppes de graines de soja concassées telles que définies dans l'une quelconque des revendications 1 à 4 ;
(b) écailler les chairs obtenues à l'étape (a) pour obtenir une épaisseur d'écaille voulue ;
(c) dénaturer à la chaleur les écailles obtenues à l'étape (b) pour obtenir un indice de solubilité de l'azote voulu ; et
(d) broyer les écailles dénaturées de l'étape (c) pour obtenir une taille de particules voulue.

8. Procédé pour fabriquer un produit de protéines de soja dégraissé, comportant les étapes consistant à :
(a) retirer les chairs des enveloppes de graines de soja concassées telles que définies dans l'une quelconque des revendications 1 à 4 ;
(b) écailler les chairs obtenues à l'étape (a) pour obtenir une épaisseur d'écaille voulue ;
(c) mettre les écailles non dégraissées obtenues à l'étape (b) en contact avec un solvant pour extraire de l'huile des écailles jusqu'à un niveau de teneur voulu ;
(d) dénaturer à la chaleur les écailles dégraissées obtenues à l'étape (c) pour obtenir une solubilité d'azote voulue ; et
(e) broyer les écailles dégraissées, dénaturées obtenues à l'étape (d) pour obtenir une taille de particules voulue.

9. Procédé pour fabriquer un produit de concentré de protéines de soja, comportant les étapes consistant à :
(a) retirer les chairs des enveloppes de graines de soja concassées telles que définies dans l'une quelconque des revendications 1 à 4 ;
(b) écailler les chairs obtenues à l'étape (a) pour obtenir une épaisseur d'écaille voulue ;
(c) mettre les écailles non dégraissées obtenues à l'étape (b) en contact avec un premier solvant pour extraire de l'huile des écailles jusqu'à un niveau de teneur voulu ;
(d) mettre les écailles dégraissées obtenues à l'étape (c) en contact avec un second solvant pour obtenir un produit de concentré de protéines de soja présentant une teneur en protéines (6,25 x N) non inférieure à 65 % (base sèche).

10. Procédé selon la revendication 9, dans lequel le second solvant est une solution aqueuse alcoolique de 55 à 90 % et dans lequel le produit de concentré de protéines de soja obtenu à l'étape (d) a une teneur en protéines (6,25 x N) non inférieure à 70 % (base séche).

11. Procédé selon la revendication 9, dans lequel le second solvant est une solution acide ayant un pH de 4 à 5.

12. Procédé pour fabriquer un produit d'isolat isoélectrique de protéines de soja, comportant les étapes consistant à :
(a) retirer les chairs des enveloppes de graines de soja concassées telles que définies dans l'une quelconque des revendications 1 à 4 ;
(b) écailler les chairs obtenues à l'étape (a) pour obtenir une épaisseur d'écaille voulue ;
(c) mettre les écailles non dégraissées obtenues à l'étape (b) en contact avec un premier solvant pour extraire de l'huile des écailles jusqu'à un niveau de teneur voulu ;
(d) mettre les écailles dégraissées obtenues à l'étape (c) en contact avec une solution aqueuse ayant un pH de 8 à 9 ;
(e) séparer les fractions soluble et insoluble du produit de l'étape (d) par des moyens physiques ;
(f) ajuster le pH de la fraction soluble obtenue à l'étape (e) pour obtenir un précipité de protéines ;
(g) séparer le précipité de protéines de l'étape (f) de la fraction soluble par des moyens physiques pour obtenir un isolat de protéines de soja ;
(h) laver le produit de l'étape (g) ; et (i) sécher par dispersion le produit lavé de l'étape (h) pour obtenir un produit d'isolat isoélectrique de protéines de soja.

13. Procédé selon la revendication 12 comportant en outre le mélange du produit d'isolat isoélectrique de protéines de soja obtenu à l'étape (i) avec suffisamment d'alcali pour augmenter la solubilité du produit jusqu'à un niveau voulu.

14. Produit de protéines de soja fabriqué à partir d'une graine de soja telle que définie dans l'une quelconque des revendications 1 à 4, ledit produit étant du lait de soja présentant une teneur en stachyose inférieure à 45 µmoles/g (tel quel).

15. Produit de protéines de soja dégraissé, non dénaturé fabriqué à partir d'une graine de soja telle que définie dans l'une quelconque des revendications 1 à 4 présentant une teneur en stachyose inférieure à 45 µmoles/g (tel quel).

16. Produit de protéines de soja thermiquement traité, désolvanté et grillé, fabriqué à partir d'une graine de soja telle que définie dans l'une quelconque des revendications 1 à 4, présentant une teneur en stachyose inférieure à 45 µmoles/g (tel quel) ; et présentant une teneur énergétique métabolisable réelle supérieure à 2 850 kcal/kg (base sèche).

17. Produit de protéines de soja thermiquement traité, dégraissé, rapidement désolvanté, fabriqué à partir d'une graine de soja telle que définie dans l'une quelconque des revendications 1 à 4, présentant une teneur en stachyose inférieure à 45 µmoles/g (tel quel).

18. Produit de concentré de protéines de soja présentant une teneur en protéines (6,5 x N) non inférieure à 65 % (base sèche) produit par le procédé comportant les étapes consistant à :
(a) retirer les chairs des enveloppes de graines de soja concassées telles que définies dans l'une quelconque des revendications 1 à 4 ;
(b) écailler les chairs obtenues à l'étape (a) pour obtenir une épaisseur d'écaille voulue ;
(c) mettre les écailles non dégraissées obtenues à l'étape (b) en contact avec un premier solvant pour extraire de l'huile des écailles jusqu'à un niveau de teneur voulu ;
(d) mettre les écailles dégraissées obtenues à l'étape (c) en contact avec un second solvant pour obtenir un produit de concentré de protéines de soja présentant une teneur en protéines (6,25 x N) non inférieure à 65 % (base sèche).

19. Produit alimentaire pour animaux ayant un taux d'inclusion de graines de soja entre 25 et 41 % et une teneur totale en stachyose inférieure à 10 µmoles/g (base sèche), dans lequel ladite graine de soja est telle que définie dans l'une quelconque des revendications 1 à 4.

20. Procédé pour produire un produit de protéines de soja présentant une teneur réduite en stachyose, comportant les étapes consistant à :
(a) croiser une lignée de graine de soja élite du point de vue agronomique avec la lignée de graine de soja mutante LR28 ou LR484 possédant un génotype qui confère un phénotype héritable présentant une teneur en stachyose dans la semence inférieure à 30 µmoles/g (sur la base de semence non séchée) ;
(b) cribler la semence de plantes de la descendance obtenue à l'étape (a) pour qu'elle ait une teneur en stachyose dans la semence inférieure à 30 µmoles/g (sur la base de semence non séchée) ; et
(c) transformer la semence sélectionnée à l'étape (b) pour obtenir le produit de protéines de soja voulu.

21. Procédé pour utiliser une graine de soja possédant un génotype au locus Stc1 qui confère un phénotype présentant une teneur en stachyose dans la semence inférieure à 30 µmoles/g (sur la base de semence non séchée) pour produire des lignées de descendance, le procédé comportant les étapes consistant à :
(a) croiser une plante de soja comportant un allèle stc1x avec un soja parent élite du point de vue agronomique qui ne comporte pas ledit allèle, pour produire un hybride F1 ;
(b) auto-polliniser l'hybride F1 pendant au moins une génération ; et
(c) identifier la descendance de l'étape (b) homozygote pour le gène stc1x et capable de produire une semence ayant présentant teneur en stachyose inférieure à 30 µmoles/g (sur la base de semence non séchée).

22. Procédé selon la revendication 21 incluant les étapes d'obtention de ladite plante de soja comportant un allèle stc1x par mutagénèse et sélection.

23. Procédé selon la revendication 21, dans lequel est identifiée la descendance qui est capable de produire une semence présentant une teneur en stachyose inférieure à 15 µmoles/g (sur la base de semence non séchée).
